# EUROPEAN PATENT SPECIFICATION

(11) **EP 3 277 296 B1**
(45) Date of publication and mention of the grant of the patent: **11.03.2020**
(21) Application number: 16723519.1
(22) Date of filing: 01.04.2016
(51) Int. Cl.: A61K 35/28, G01N 33/50, A61K 35/50, A61P 37/00, A61P 1/00

(54) **COMPOSITION FOR USE IN TREATING CELIAC DISEASE**
ZUSAMMENSETZUNG ZUR BEHANDLUNG VON ZÖLIAKIE
COMPOSITION POUR LE TRAITEMENT DE LA MALADIE COELIAQUE

(30) Priority: 02.04.2015 IT RM20150136
(43) Date of publication of application: 07.02.2018
(73) Proprietor: Stegi-Ra Trust, 6900 Paradiso (CH)
(72) Inventor: CICCOCIOPPO, Rachele, 27100 Pavia (IT)
(74) Representative: Galassi, Alessandro
(86) International application number: PCT/IB2016/051872
(87) International publication number: WO 2016/157142

(56) References cited:
- WO-A1-2008/146992
- WO-A1-2009/105044
- WO-A1-2013/169202
- WO-A1-2014/013029
- WO-A1-2014/087435
- WO-A1-2014/125277
- WO-A2-2014/191978
- WO-A2-2015/016761
- MAUMUS MARIE ET AL: "Mesenchymal stem cells in regenerative medicine applied to rheumatic diseases: Role of secretome and exosomes", BIOCHIMIE, vol. 95, no. 12, 16 May 2013 (2013-05-16), pages 2229-2234, XP028768026, ISSN: 0300-9084, DOI: 10.1016/J.BIOCHI.2013.04.017
- LAVOIE JESSIE R ET AL: "Uncovering the secretes of mesenchymal stem cells", BIOCHIMIE, vol. 95, no. 12, 28 June 2013 (2013-06-28) , pages 2212-2221, XP028768029, ISSN: 0300-9084, DOI: 10.1016/J.BIOCHI.2013.06.017

## Description

### Field of the invention

The present invention concerns the field of the preparates for treating of diseases, and in detail concerns a composition for treating of the celiac disease.

The present invention further concerns a method for the preparation of the said composition for treating of the celiac disease.

The present disclosure concerns finally a test of analysis of presence of gliadin T specific lymphocytes, that is performed in vitro in association to the therapy of the celiac disease performed with the composition object of the present invention.

### Background art

The celiac disease is an autoimmune enteropathy triggered and sustained by the ingestion of gluten with the diet in genetically predisposed individuals. Said disease is caused by an abnormal immune response towards antigens both exogen like the components of the gluten, and endogen, like the tissue transglutaminase, that has the small intestine as a target organ.

It has a high prevalence in the general world population and its incidence is increasing not only since an increasing number of patients is correctly diagnosed, but also by virtue of a real diffusion of the disease. As a consequence, the celiac disease should reach epidemic levels in the forthcoming future. Anyway, also if the diagnostic tests are very accurate, many cases still rest not diagnosed due to the variability of the clinical picture that goes from an asymptomatic or oligo symptomatic condition of most part of the patients, to a serious malabsorption syndrome. Notwithstanding the great progresses performed in the last ten years into comprehending the fine mechanisms which are responsible of the intestinal lesions, the therapy is still based on the gluten-free diet, as originally proposed by the Dutch pediatrician, doctor Willem Dicke. Also if this treatment guarantees the recuperation of the clinical picture as well as the intestinal damages in almost all patients, it influences the quality of life, therefore generating a condition of chronic stress, forcing patients to a sort of social segregation. Furthermore, the gluten-free diet does not protect the patients from developing complications, like the refractory celiac disease and the T-cells lymphoma associated to enteropathy. These last, also even if very rare, represent a challenge, since there are no available standardized therapies and the prognosis is unfavorable. Furthermore, the possibility of experimenting new therapeutic strategies is compromised by the lack of animal models, and on the other side, any proposed drug implies a series of potential adverse effects that exclude its use as substitution of the gluten-free diet regime. Between the different approaches under examination, none has marked a certain effectiveness once tested in vivo, probably because the experimented molecules are provided with a single target, while the immunologic cascade which is responsible of the tissue damage is complex and redundant. As a result, the arrival of a safe and effective therapy for the celiac disease is still an unsatisfied need.

The stem cells have always fascinated doctors and researchers for their potential therapeutic applications. Anyway, considering the ethical limitations about the use of embryonal stem cells, the invasivity and the risks which are associated to the transplant of hematopoietic stem cells, only the mesenchymal stem cells (MSCs) are considered being the best candidate for clinical use, most of all in conditions that do not threaten the life of the patients. Originally isolated from bone marrow, and subsequently in different adult and fetal tissues, this cellular population has at first attracted the interest due to the easiness of ex vivo isolation and expansion, the capacity of differentiation towards different cellular lines, and the migration towards active phlogosis sites. Recently it has been recognized a powerful action over all the cells involved in the immune response, with the final effect of dampening the inflammation, favoring the expansion of the regulatory T-cells under populations, and the repair of damaged tissues, among which also the intestine. Furthermore, the substantial absence of immunogenicity renders them an interesting therapeutic instrument, since it is not necessary to perform a preventive regime of immune-ablative conditioning for their transplant.

To this end, the MSCs are been already safely and effectively used as an extreme therapy in the acute forms of transplant steroid-refractory disease against the host and of autoimmune enteropathy (Ciccocioppo R, Russo ML, Bernardo ME, Biagi F, Catenacci L, Avanzini MA, Alvisi C, Vanoli A, Manca R, Luinetti OR, Locatelli F, Corazza GR. Mesenchymal stromal cell infusions as rescue therapy for corticosteroid-refractory adult autoimmune enteropathy. Mayo Clinic Proceedings 2012; 87: 909-14), two immune-mediated conditions wherein the intestinal lesions are similar to those of the celiac disease. Furthermore, the MSCs have resulted effective both if administered intravenously or directly in the fistulas of patients affected by refractory Chrohn's disease (Ciccocioppo R, Bernardo ME, Sgarella A, Maccario R, Avanzini MA, Ubezio C, Minelli A, Alvisi C, Vanoli A, Calliada F, Dionigi P, Perotti C, Locatelli F Corazza GR. Autologous bone marrow-derived mesenchymal stromal cells in the treatment of fistulizing Crohn's disease. Gut 2011; 60: 788-98.), another intestinal disease triggered and sustained by an abnormal inflammatory response towards the antigens of the intestinal gut, that develops in genetically predisposed individuals.

Finally, passing to consider which source of MSCs seems to be the more suitable to the clinical application, apparently no relevant biologic differences have been found between the different tissues of origin. Anyway, the potential limitation of the donors and the invasivity of the procedure of collection which is necessary when bone marrow and adipose tissue are used, have rose the need of using alternative sources with unlimited donors and ease of access, like for example the tissues which are released at the moment of the birth, like blood of umbilical cord, the Wharton gelatin, the amniotic liquid, amnion and placenta. This last should have enormous proliferative and regenerative features, together with an high tolerogenic activity, since it generates the status of fetal immuno-privilege which is necessary to guarantee the regular developing of the pregnancy.

Anyway, it exists a series of limitations to the use of intact cells. A first problem is represented by the influence of the mucosa micro environment and the hierarchy with which the MSCs exert their in vivo action in this specific pathologic setting.

**Preliminary observation about the present invention, identified by the applicant.**

The applicant has recently shown that the MSCs have a powerful effect in vitro suitable of suppressing the reactivity to gluten and induce a tolerogenic response for the T-lymphocites of the patients, the principal cellular population responsible of the intestinal damage (Ciccocioppo R, Camarca A, Cangemi GC, Radano G, Vitale S, Betti E, Ferrari D, Visai L, Strada E, Badulli C, Locatelli F, Klersy C, Gianfrani C, Corazza GR. Tolerogenic effect of mesenchymal stromal cells on gliadin-specific T lymphocytes in celiac disease. Cytotherapy; 2014: 16: 1080-91). Furthermore, the MSCs shall be induced by the immune cells activated before fully developing their tolerogenic and anti-inflammatory action, and said induction could not take place or be subjected to an in vivo adaptation. A further question concerns the possibility that the infused cells could lose their functions, or simply could not be capable of reaching the target site. Today, the most common way of administration for MSCs is the intravenous one, and various evidences demonstrated that the MSCs injected in such a way rest trapped in the lungs at their first passage due to their size. Finally, the possibility of triggering a rejection reaction, or of being killed when injected in an immunocompetent host represents a further criterion for limiting their clinical use. Further problems relate to their survival once injected, the capacity of keeping their function inside the host and the risk of carcinogenicity.

These unpredictable results rise doubts about the possibility of controlling the effect of the MSCs when being employed into the therapy in complex immune-pathogenesis conditions. Today, the effects benefits of the MSCs have been attributed principally to the cell-cell interaction and to the release of a wide range of bio active molecules, than to the direct substitution or the routing to the damaged tissue, and recent studies show that the vesicles released are the operative paracrine agent.

This hypothesis opens new perspectives finalized to the development of acellular strategies based on the use of vesicles derived from the MSCs as safe and potentially more advantageous approach in alternative to the traditional cellular therapy. Finally, the use of vesicles allows for avoiding the waste of time which is necessary for isolating and expanding the MSCs, that requires generally from four to six weeks in order to obtain a sufficient amount of cells necessary for the therapeutic purposes, and estimated approximately in 2.0 x 10⁶ cells/kg of body weight.

The scope of the present invention is therefore to describe a composition comprising vesicles which are released by the MSCs, in particular suitable for being used into the therapy of the celiac disease; said composition is therefore targeted to overcome the aforementioned drawbacks.

The scope of the present invention is furthermore to describe a method of production of the said composition comprising vesicles released by the MSCs.

### Summary of the invention

The present invention is defined by the claims. Any subject matter falling outside the scope of the claims is provided for information only.

Any references in the description to methods of treatment refer to the compounds, pharmaceutical compositions, and medicaments of the present invention for use in a method for treatment of the human or animal body by therapy.

The present invention comprises therefore a method of preparation of a composition comprising extracellular vesicles (further described in the course of the present description with the term EV) from MSCs, said method being characterized in that it comprises the following steps:
- a step of extraction of extracellular vesicles from MSCs, said extracellular vesicles being derived from a single sample of chorion (intended as "unique" for each patient) in advance separated from the placenta in a step of separation chorion-placenta and
- a step of sterilization and elimination of contaminating proteins from a semi-finished fluid containing said extracellular vesicles, wherein said step of sterilization and elimination of contaminating proteins comprises at least a first step of ultracentrifugation of the said semi-finished fluid, and wherein
- said first step of ultracentrifugation is performed following of a phase of filtration of said semi-finished fluid.

According to an aspect of the present invention, said method is characterized in that it comprises a second step of ultracentrifugation of the said semi-finished fluid, wherein said second step of ultracentrifugation is performed after said first step of ultracentrifugation following of an intermediate step of washing, performed between said first and said second step of ultracentrifugation and is performed on a growth medium without serum.

In a further aspect of the present invention, said method is characterized in that it comprises a plurality of steps of centrifugation, being performed before said first and/or second step of ultracentrifugation, wherein said plurality of steps of centrifugation separate cellular detritus and/or dead cells from said extracellular vesicles, and wherein each of the said steps of centrifugation is performed for an amount of time equal or greater than 10 minutes, and wherein said semi-finished fluid comprises surfloating elements following said plurality of steps of centrifugation.

Advantageously, said plurality of steps of centrifugation takes place on a surfloating portion (supernatant) of a further semi-finished comprising said MSCs, said further semi-finished being preventively incubated for a multi-day period of time and being undergone to a treatment of increase of the immunologic properties of said MSCs .

Advantageously, said treatment of increase of the immunologic properties of said MSCs comprises a step of washing by means of PBS followed by a treatment with human recombinant interferon-γ.

In a further aspect of the present invention, the surfloating which are necessary for preparing the extracellular vesicles, that represent said further semi-finished, are subjected to a step of changing of a growth medium from a first Chang C type to a second Dulbecco Modified Eagle Medium type, and wherein said second type of growth medium is supplemented with a predefined percentage of Exo-FBSTM Depleted Exosome.

Advantageously, a plurality of samples of the said chorion is subjected to a step of digestion performed in two times, at first by means of using pronase and subsequently of collagenase, in percentage preferably and respectively of 0,125 g/L and 0,1g/L.

In detail, said step of digestion performed in two times is blocked by means of the addition of a balanced saline solution and subsequently through a step of centrifugation; and wherein following of the block of the said second time of the step of digestion each pellet produced from said step of digestion is newly suspended in a Chang Medium C growth medium supplemented with bovine fetal serum deactivated by heat and with gentamicin for then being subjected to a subsequent step of incubation in humidified atmosphere. Advantageously, furthermore, following of the said step of incubation in humidified atmosphere, the growth medium is substituted and is performed a step of enzymatic digestion after having removed the growth medium and having added trypsin. Advantageously, said step of digestion is blocked by means of the addition of a predefined percentage of Hank's balanced salt solution (HBSS) and by means of a centrifugation following which the pellet is suspended in a growth medium and is performed a control of the vitality of the cells.

Advantageously, said extracellular vesicles obtained following the two steps of ultracentrifugation are made adhere to sulphate latex beads and subsequently subjected to cytofluorimetric analysis.

The method described in the present invention comprises advantageously a step of adipogenic differentiation of the said MSCs and a step of osteogenic differentiation and wherein said step of adipogenic differentiation comprising in turn:
- a step of seeding (of a plurality of cells in a specific growth medium);
- a subsequent step of adding of the said growth medium specific with a Mesencult medium and a supplement of said Mesencult medium

In detail, said step of adipogenic and osteogenic differentiation is followed by a step of evaluation of said differentiation comprising a coloring with lysochromic nitrocomposition or with alizarin respectively, and a subsequent plating and cultivation up to 100% or 50% of confluence, respectively, in a growth medium specific for MSCs in humidified atmosphere. Advantageously, said method comprises a step of analysis of the expression of the molecules of surface of the MSCs by means of flow cytofluorimetry.

Advantageously, said method comprises furthermore a step of analysis of genomic stability wherein a sample of cells of each lot of MSCs is subjected to a karyotype analysis comprising a step of incubation performed in advance respective to a step of banding. The method of preparation of the composition EV according to the present invention comprises advantageously a step of microbiologic test for verifying the absence of anaerobic and aerobic bacteria, endotoxins and micoplasmas.

The method of preparation of the said composition of EV comprises advantageously also a step of evaluation of the composition of the said preparation performed in electronic microscopy by means of a first step of suspension of a pellet in paraformaldehyde followed by a subsequent step of resting in dry atmosphere for allowing the absorption of the membrane, said step of resting being furthermore followed by a step of contrast of the samples with uranyl-oxalate and subsequent incorporation of said samples in a mixture of uranyl acetate and methylcellulose.

The method object of the present invention further comprises a step of analysis in vitro of the immunologic response to said EV, comprising a step of collecting samples of EV of a same lot that are incubated together with mono-nucleated peripheral blood cells (PBMC) in advance isolated from a patient affected by celiac disease by means of centrifugation and subsequent recuperation (280) with subsequent suspension in growth medium added with deactivated human serum and with antibiotic preferably comprising penicillin and/or streptomycin, and wherein the cellular vitality is determined by means of a test of exclusion with trypan blue. From said cellular population, the T- lymphocites are therefore separated by means of immuno-magnetic sorting in the cytofluorimeter.

In said method is furthermore comprised a pre-infusion test comprising a step of seeding of the T- lymphocytes in a plurality of retention means like the Petri plates or equivalent liquid containers within which PBMCs in advance irradiated and used as cells having antigen are contained, and a step of subsequent addition of said EV within said retention means; said step of addition is furthermore followed by a step of incubation of said lymphocytes or by a step of stimulation with mitogen polyclonal phytohaemoagglutinin; said method comprising further a step of incubation and of subsequent collection and freezing of the surfloating collected from said retention means for a subsequent determination of level of interferon-y, and comprising furthermore a step of pulsation of the cells T with tritiated thymidine (³H thymidine).

According to the present invention is furthermore realized an injectable preparation of EV for use in for treating of the celiac disease, obtained by means of the method previously described. According to the present invention is furthermore performed a test of analysis of presence of gliadin-specific T- lymphocytes, to the end of evaluating the therapeutic effectiveness of the preparation according to the present invention.

In detail, said test comprises a phase of isolation of PBMC by means of a centrifugation followed by washing in PBS, and comprises furthermore a phase of control comprising a positive control and a negative control , wherein said positive control, targeted to show the presence of the said gliadin-specific T- lymphocytes, comprises a step of counting of spot-forming cells (SFC) in increment in said peripheral blood in case the PBMC are treated with an antigenic preparation of gliadin, and wherein said negative control comprises a step of counting of the increment of SFC in the said PBMC in case are incubated only with growth medium.

Said test of presence of gliadin-T specific lymphocyte providing positive result in case a ratio between: increment of SFC in said positive control and increment of SFC in said negative control is greater than 3.

Advantageously, said antigenic preparation of gliadin used for said test is in concentration of 50 mg/ml.

### Description of the figures

The invention will be hereinafter described referring to a preferred embodiment, with the support of annexed figures wherein:
- figure 1 shows a detail of extraction of an extracellular vesicles from MSC;
- figure 2 shows a detailed scheme of coding of the placenta;
- figure 3 shows a first diagram of process for realizing of a preparation for treating of the celiac disease;

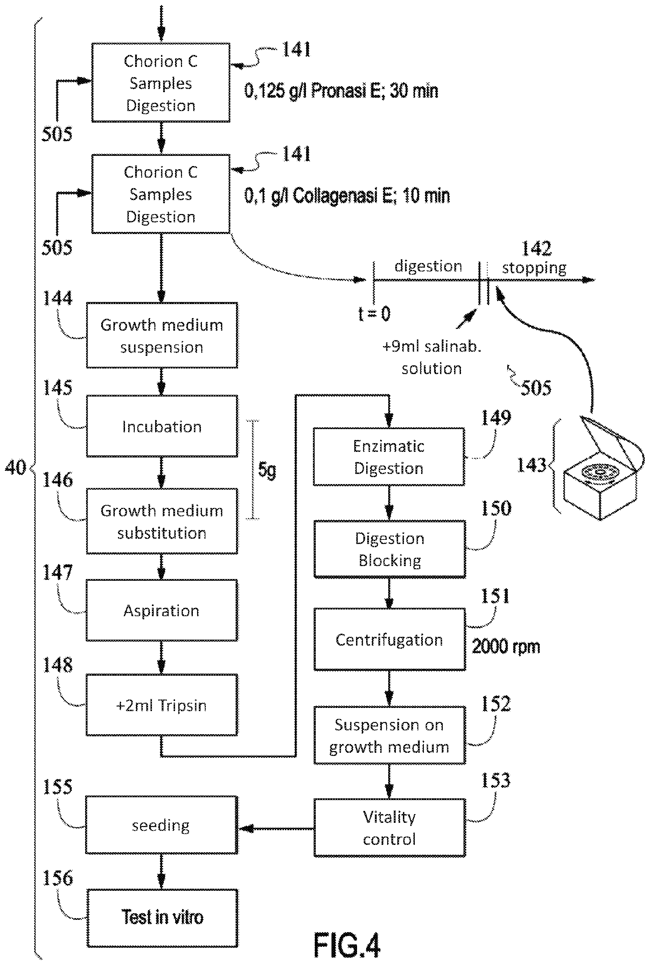

- figure 4 shows a second diagram of process for realizing of a preparation for treating of the celiac disease;
- figure 5 and figure 6 show respectively a diagram showing a part of the process of adding and incubation of the MSC and of morphological analysis thereof;
- figure 7 shows a diagram reporting a process of ultracentrifugation in more stages, necessary for producing the injectable preparation object of the present invention;

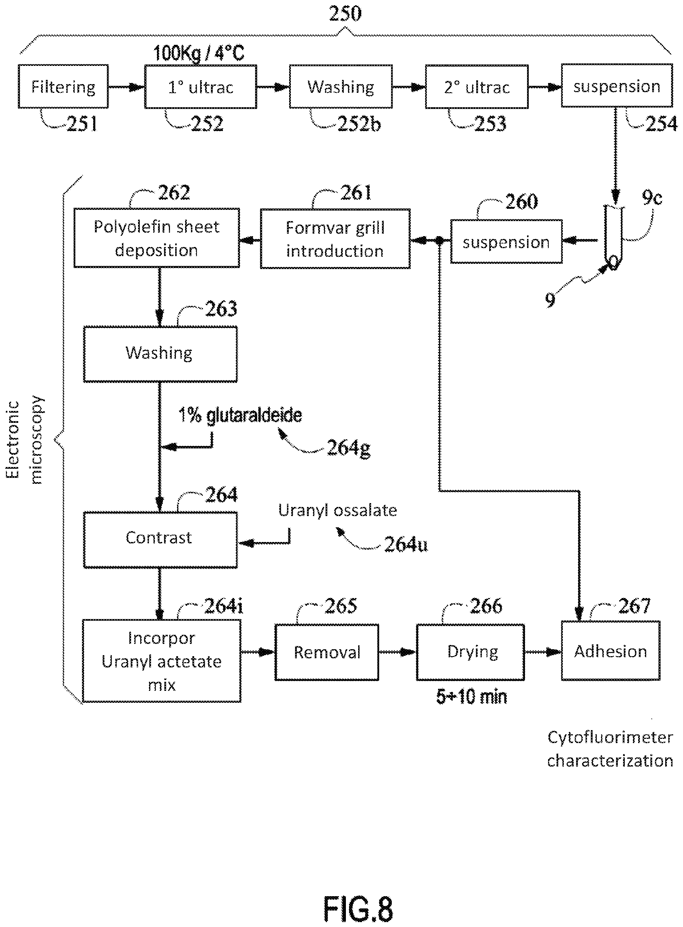

- figure 8 shows a further process of processing of an intermediate preparation and its morphological characterization to the end of obtaining the injectable preparation object of the present invention; and

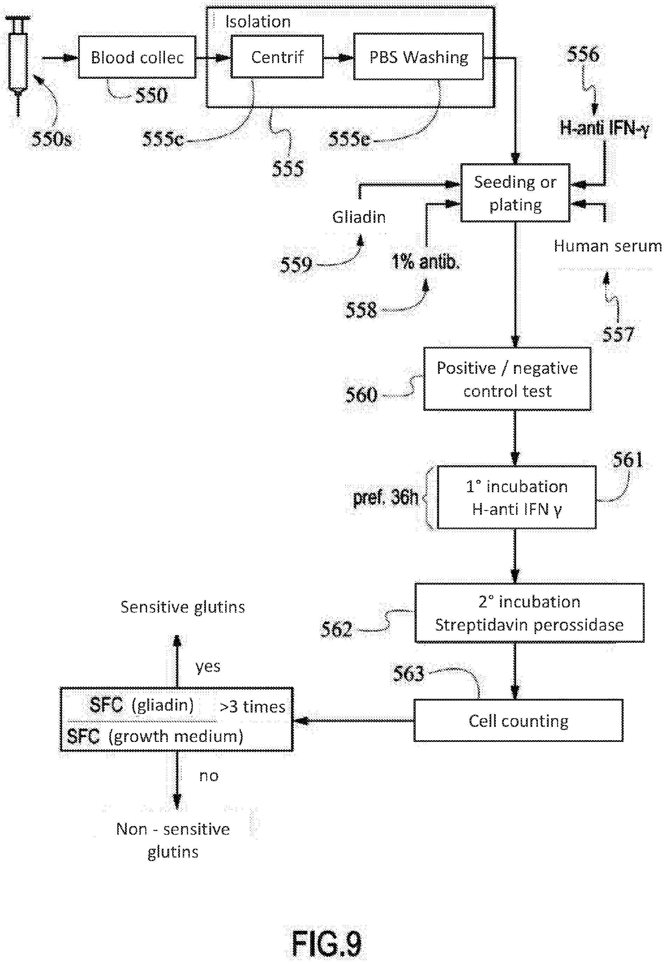

- figure 9 shows a diagram reporting a test process for verifying the presence of gliadin-T specific lymphocytes in peripheral blood collected from patients subjected or to be subjected to a cure of the celiac disease by means of the preparation object of the present invention.
- figure 10 shows a diagram of a chronological distribution of a clinical course of a patient affected by refractory celiac disease following of the treatment with mesenchymal cells;
- figure 11 shows a diagram of the interleukin (THE)-15 / (THE)-15Ra levels in the mucosa of said patient during the treatment with mesenchymal cells, respective to the levels of patients with active celiac disease, treated and objects of control.
- figure 12 shows a characterization of the cells mesenchymal of marrow of said patient.

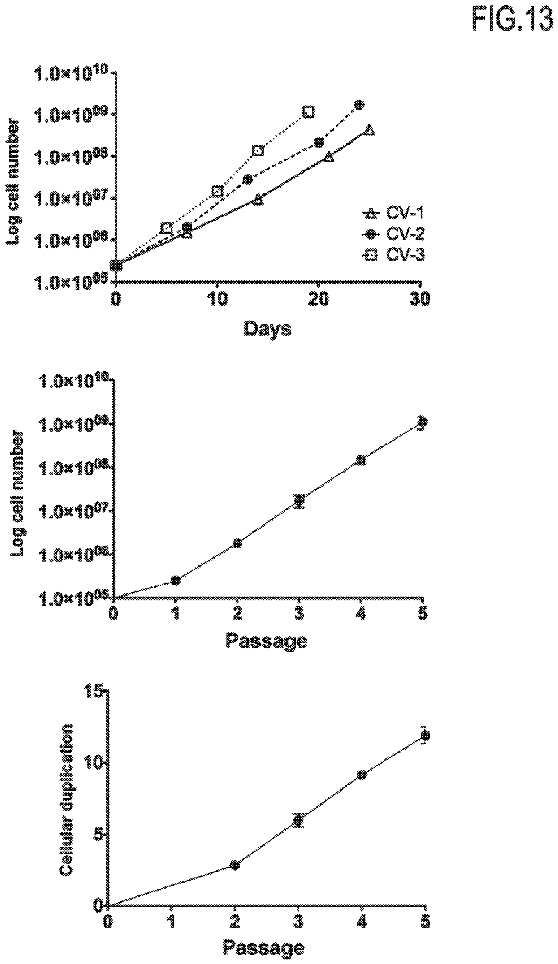

- the figure 13-15 show bi-dimensional diagrams of the trend respectively of growth mesenchymal cells from chorion, of samples of proliferation of gliadin-T specific lymphocytes and production of interferon-y following of antigenic re-stimulation, in presence and absence of EV isolated both from mesenchymal cells of marrow (BM-MSC) stimulated and non-stimulated and still from cells mesenchymal of chorion (C-MSC) stimulated and non-stimulated, at different ratios.

### Detailed description of the invention

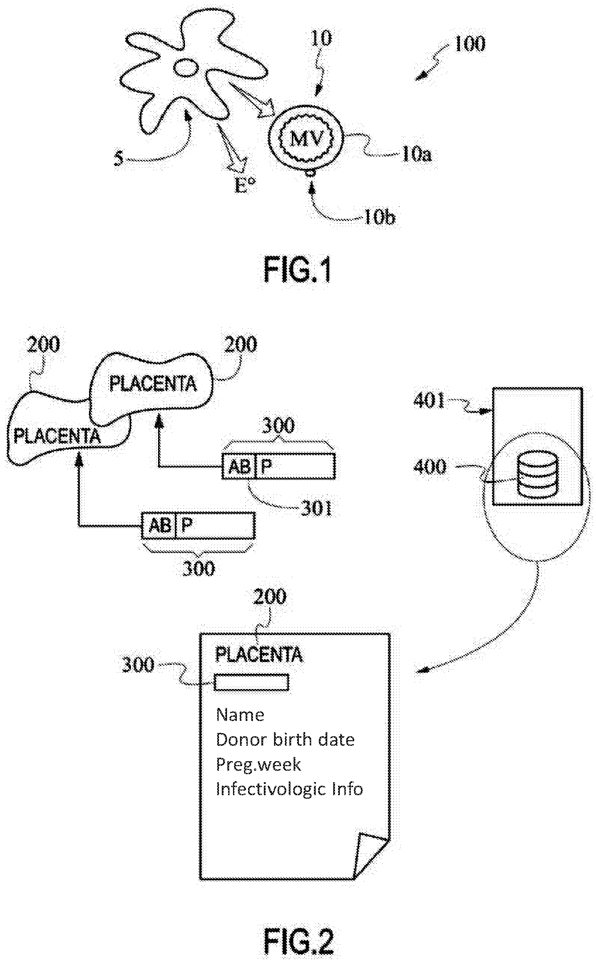

Referring to the annexed figures, and in detail to figure 1, with the reference number 100 is shown in its integrity a composition for treating of the celiac disease. The composition 100 comprises in detail of the extracellular vesicles(EVs 10).

The extracellular vesicles (EVs 10) are organelles surrounded by membranes 10a released by intact MSCs 5 and containing all the active molecules which are responsible of the their biologic effects. They comprise the exosomes (E) that are released by means of exocytosis and the micro vesicles that gem from the cellular surface. In particular, the exosomes are formed at intracellular level by the introflexion of the limiting membrane of the endoplasmic compartments, therefore generating the endosomes containing vesicles, called tardive endosomes or multi vesicular bodies (MVB). The MVBs finally merge with the plasmatic membrane, therefore liberating the internal vesicles(that is the exosomes) in the extracellular space. Given their small sizes (50-120 nm), the exosomes can be viewed only by means of electron microscope; therefore, the evaluation of the purity of said preparates, as well as their characterization should be always determined by means of electron microscopy.

The exosomes tend to be homogeneous in size and are released by means of a mechanism of p53-mediated exocytosis, that depends on the activation of the cytoskeleton, but is independent of the flow of calcium. The micro vesicles, also known as ectosomes or micro particles, directly come from the blebbing of the plasmatic membrane. They are more heterogeneous in size that goes from 100nm to 1 micron, and their release depends on system of the calpain, on the re-organization of the cytoskeleton and of the concentration of intracellular calcium. The calcium ions, in fact, are those which are responsible of the changing into the distribution of phospholipids of the membrane that determinate the formation of the vesicles.

There is a further type of extracellular vesicles, of size greater of 1 micron, named apoptotic bodies, deriving from cells into the final phase of the program of cellular death where there are often DNA residuals. Anyway, a certain confusion on the exact definition of exosomes and micro vesicles is present in literature, since some researchers use these wording according the rigorous definitions, while other in an interchangeable way. In particular, the term micro vesicles has been often used for indicating both the types of vesicles, independently from their intracellular origin. Recently, the International Society on the Extracellular Vesicles has recommended of using the wording "extracellular vesicles" as a generic term for all the types of vesicles which are present in the extracellular space, among which the exosomes, the micro vesicles and the apoptotic bodies. Following this recommendation, we will use the wording EV in the whole description, even though the reference is principally made on exosomes. The EVs 10 represent a potential therapeutic instrument, thanks to their capacity of influencing the target cells by means of the release of bioactive substances. In particular, they are capable of mediating the communication between cells through the horizontal transfer of different membrane and cytoplasmic molecules, like the proteins, lipids, mRNA, microRNAs, and nucleic acids. Following of their adhesion which is mediated by the receptors and their internalization, the target cells undergo a great variety of phenotype and epigenic changing, therefore being reprogrammed. It can be plausible therefore that the EVs 10 derived from the MSCs 5 can induce immunologic tolerance transferring their content. The role of the Evs 10 as vehicles of immunologic and regenerative effects has been demonstrated in various works in vitro, and in experimental models in vivo.

To this end, the EVs 10 could represent a therapeutic instrument safer and more reproducible with respect to the MSCs 5. The application of the exosomes, in fact, offers a series of advantages respective to that of the MSCs themselves, since they are more stable, induce stronger signals, and their preservation and use are easier. Furthermore, the EVs 10 do not present any risk of aneuploidy or rejection as in contrast the MSCs have following an infusion in vivo in an allogenic setting.

The exosomes, furthermore, are not auto replicating and thanks to their small size they can be sterilized by means of filtration.

Therefore, the norms to be followed for their use on therapeutic purposes are less complex respective to those which rule the manipulation of intact cells. To this end, the possibility of curing the acute and refractory to standard therapy disease from transplant against the host by means of the use of macrovesicles derived from MSCs 5 has recently been performed successfully.

For a better comprehension of the present description, the meaning of some definitions used in the context of the present patent application are hereinafter enunciated. In detail:
- Placenta.
   The placenta is the organ with a circular and flattened shape, contained in the uterus of pregnant mammals, devoted to the metabolic exchanges between the mother and fetus, and constituted by a maternal part, or decidual, and of a fetal part, which is the chorion.
- Amnion.
   The most internal of the two membranes that enclose the mammal embryo.
- Chorion.
   The external membrane that surrounds an embryo of the mammal.
- Stem cells.
   They are non-differentiated cells that are arranged in differenced tissues, provided with a huge proliferative capacity, of auto-renewing and of differentiation in specialized cells. The mesenchymal stem cells of the present patent derive from placenta's chorion.
- Isolation This procedure indicates that the cells to which we refer are not in their natural environment, since they have been separated from their original tissue.
- Expansion.
   This procedure refers to the capacity of a population of cells of increasing their critical mass in appropriate conditions of culture.
- Differentiation.
   This term refers to the formation of cells that express markers known for being associated with cells specialized and close to become terminal since not being capable of further division or differentiation.
- Immunophenotype
   With 'positive' shall be meant that a marker is expressed in a cell. For being considered expressed, a marker shall be at a detectable level. With 'detectable level' shall be meant that the marker can be detected using a standard methodology like the flow or immunohistochemical cytometry. A cell is considered 'negative' if the expression of that specific marker cannot be detected.
- Extracellular vesicles.
   Those cellular elements are organelles covered by a membrane and released by intact cells, containing bioactive molecules that mediate fully their effects.
- Growth medium.
   Refers to any substance or preparation used for cultivating living cells. The base medium generally serves for the preparation of a more complex means, by means of addition of serum, tampons, growth factors and antibiotic. In this means, the cells can be cultivated in incubators with controlled levels of CO₂ and O₂, at 37° C.
- Surfloating (supernatant),
   It indicates the liquid staying over a solid residual after the centrifugation.
- Pellet.
   It indicates the solid residual at the bottom of the tube after the centrifugation.
- Lot.
   The amount of EVs 10 that derives from a single sample of chorion and that shall be homogeneous. The lot number is the name of the assembly of numbers and/or letters of the alphabet that allow the identification thereof.
- Vehicle.
   The sodium chloride solution, comprised in a percentage between [0,8-1,0]% and preferably at 0,9%, together with human albumin; in said solution the EVs 10 are suspended before the therapeutic use. The percentage of the solution of sodium chloride should therefore be iso-osmolar with the human blood, and the aforementioned percentages allow advantageously reducing the risk that said solution could damage the cells.
- Storage.
   The method of conservation for a future use, i.e. cryo-conservation.
- infusion.
   Injection of the preparation of EVs 10 in the human body for therapeutic purpose using a peripheral vein.
- Gluten.
   The gluten (from Latin gluten: glue) is a mixture of reserve proteins which are present in the endosperm of the cereals, among which the wheat, the barley and the rye. These are classified in four classes according to their solubility: the albumins are soluble in water, the globulins in saline solution, the gliadin in alcoholic solution, while the glutens are insoluble in aqueous solution or neutral saline and ethanol. The gliadins are the most toxic and immunogenic component for celiac patients. The gluten confers elasticity to the mixture thanks to its capacity of trapping carbon dioxide, therefore contributing to make it rise and conferring a gummy consistence to the final product.
- Tissue transglutaminase.
   It is an ubiquituous enzyme whose principal function is to catalyze the covalent and irreversible bond between a residual of glutamine of a donor-glutamine protein with a residual of lysine of a glutamine-acceptor protein with the formation of a ε-(γ-glutamil)-lysine (isopeptidyl) bond. Anyway, excluding the transamidation reaction, this enzyme can also de-starch a glutamine residual in glutamic acid in presence of a low pH or when acceptor proteins are no more available. Said process implies a greater immunogenicity of the peptides of the gluten that represent its preferred substrate.
- Refractory.
   A patient that does not respond to standard therapies.
- Tolerogenic.
   The mechanism due to which a substance does not evoke a immunologic response.
- Bioactive molecules.
   Molecules as proteins, lipids, mRNA, microRNA, and nucleic acids that are responsible of the biologic effects of that specific cellular population.

Furthermore, in the course of the present description for the sake of convenience will be used the following abbreviations among which appear:
Dimethyl sulfoxide: DMSO; Dulbecco's Modified Eagle Medium: DMEM; Exosomes: E; Extracellular vesicle: EV; fetal calf serum: FCS; fetal bovine serum: FBS; fluorescein-isothiocyanate: FITC; fluorescence activated cell sorting: FACS; fold increase: FI; Good Manufacturing Practice: GMP; Hank's Balanced Salt Solution: HBSS; Human Leucocyte Antigen: HLA; interferon: IFN; mesenchymal stem cells: MSC; multivesicular body: MVB; peripheral blood mononuclear cells: PBMC; phosphate-buffered saline: PBS; phycoerythrin: PE; RNA; Roswell Park Memorial Institute: RPMI; Spot-forming cells: SFC.; stimulation index: SI.

Into the following portion of description is therefore described the process object of the present invention with which the MSCs are isolated and expanded from chorion of human placenta.

In detail, from laboratory preliminary experiments, a negative correlation between the age of the mother and the obtained number of MSCs has been observed. It has therefore been decided, as it is shown in the first step 100, of using placentas from mothers of age lower than 35 years. Furthermore, to the end of establishing the origin of the MSCs, if from the mother or from the child, in some cases placentas of pregnant women of male fetuses have been used. Furthermore, for ensuring the traceability, keeping the privacy, a second step 110 provides for assigning to every placenta 200 of a code of identification 300 automatically generated by an electronic program; in detail, said generation of said code of identification 300 takes place by means of the introduction of a alphabetical under-code 301 for each sample derived therefrom; said alphabetical under-code 301 will identify the lots of MSCs, followed by a 'P' indicating the number of passage in culture. In a database 400 in electronic format and stored on a remote server 401 are collected the following information: the name and date of birth of the donor, the gestation week, the infectivologic information relative to the results of the screening for the virus of human immunodeficiency and the hepatitis viruses B and C.

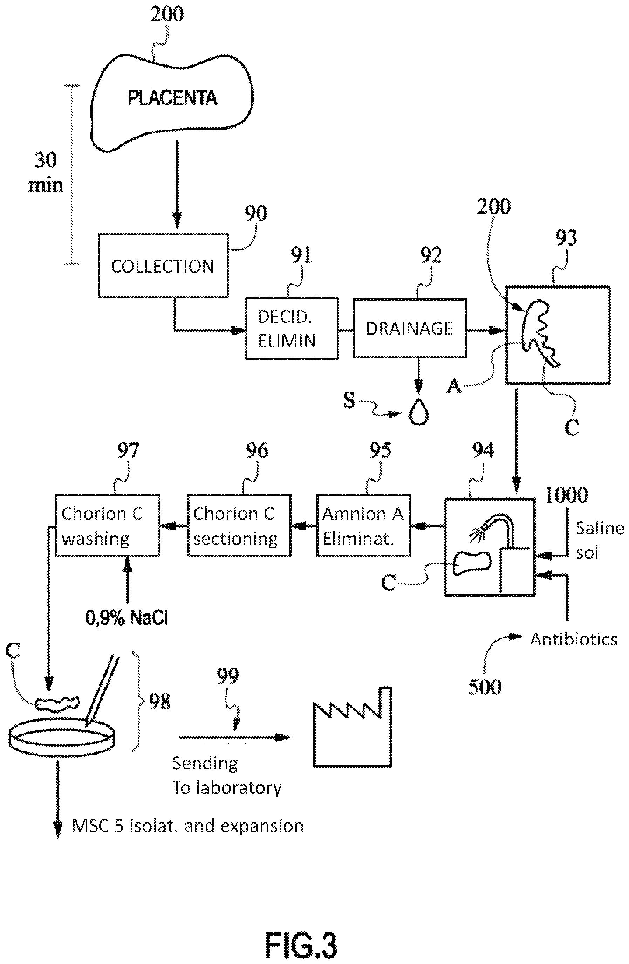

For isolating the MSCs, the human placenta at end (36a-40a gestation week), is at first collected fresh (step 90, figure 3) aseptically within 30 minutes from the birth by means of caesarean section performed in election on a sane infant in absence of potential procedural complications. The first passage 91 consists of the removal of the parietal deciduous by means of careful scraping, followed by a further step 92 of drainage of blood by the arteries and by the veins. Then (step 93, separation manual of chorion C and amnion), the amnion and the chorion are manually separated and washed (step 94) in buffered saline solution (1000) (PBS, Sigma-Aldrich, Saint Louis, MO, USA) containing antibiotic (500).

Preferably, the antibiotics 500 used for washing are 1% of penicillin/streptomycin, 0,25% gentamicin, for example and coming from Lonza Group Ltd; Basel, Switzerland. Subsequently, in a step of elimination of the amnion 95, the amnion is eliminated , while the chorion is sectioned (step 96) in a series of full thickness fragments of circa 2-3 cm³ by means of a scalpel. The samples are immediately washed (step 97) at least two and preferably three times through sequential passages in sterile physiologic solution, preferably at 0,9% of sodium chloride, using plastic containers to the end of draining the excess of blood. The washing for three times guarantees a correct elimination of the impurities from samples, that a single washing would not reach. Furthermore, the solution at 0,9% of sodium chloride advantageously allows for of obtaining a correct washing chemically speaking without damaging the sample.
every piece is then transferred (step 98) in a Petri dish containing 5 ml of Chang medium C (#C100; IrvineScientific; 2511 Daimler St. Santa Ana, CA, USA) for being immediately sent (step 99) to the laboratory and processed according to the protocol of Good Manufacturing Practice (GMP) for cellular therapies.

Then the macro process of isolation and expansion of the MSCs takes place. In said macro process, that in the annexed figures is represented with the reference number 40, the samples of chorion are digested (step 141) by means of serial incubations with 0,125 g/L of pronase and (Merck KGaA; Darmstadt, Germania) and 0,1 g/L of gluegenase (Sigma-Aldrich) a 37° C per 30 and 10 minutes, respectively.

At the end of every phase of digestion, the digestion is blocked (step 142) adding 9 ml of balanced saline solution5 05 of Hank cold (1x HBSS 24020; Gibco Life Technologies, Paisley, UK) and centrifugation (step 143) at 2000 rpm for at least 10 minutes. The stopping or blocking of the process of digestion takes place therefore two times, one for each digestion. Finally, each pellet is newly suspended (step 144) in 5 ml of growth medium that preferably is constituted by Chang Medium C (IrvineScientific) supplemented with bovine fetal serum at 20% deactivated by heat and gentamycin 1% (Lonza) in a flask T25 and incubated (step 145) in humidified atmosphere at 95% at 37° C. The complete growth medium is substituted (step 146) after 5 days, and then twice a week up to confluence (P0). At this moment, the MSCs adherent one another are separated by means of enzymatic digestion (step 149) after having removed the growth medium by means of aspiration (step 147) and adding 2 ml of trypsin (step 148) (TrypLE 12605; Gibco Life Technologies) for 10 minutes at 37° C.

The process of digestion here aforementioned is therefore blocked (step 150) adding 5 ml of balanced saline solution of Hank, also known with the acronym HBSS, of the type 1x (Gibco Life Technologies) and centrifugation (step 151) at 2000 rpm for 10 minutes. The pellet is then newly suspended (step 152) in Chang C fresh medium and the vitality of every sample cellular is valuated (step 153) by means of trypan blue test in Burker chamber. The cellular samples are in detail eliminated if the vitality is lower than al 95%.

Finally, the cells are newly seeded (step 155) to a density preferably equal to 10.000 cells/cm² (2,5 x 10⁵ cells/flask T25) ad every passage, and the sub-culture of MSCs 5 from P3 to P6 are tested "in vitro" (step 156) before their usage for the production of EVs 10. In particular, only if the MSCs respond to all the criteria for clinical use, that is, tapered morphology, absence of contamination from pathogens, vitality >95%, immunophenotype with positivity for the expression of CD73, CD90, and molecules of surface CD105 (>95%), absence of CD34, CD45, CD31 (<5%), and capacity of differentiation in adipogenic and osteogenic tissue following appropriate stimuli, will be used for producing EVs 10.

Hereinafter is described the biologic characterization of the MSCs from human chorion. At a level of morphology, a homogeneous population of adhering cells, simil-fibroblastic, is regularly observed at every passage in culture of the MSCs derived from chorion.

In contrast, in terms of immunophenotype, the typization is performed by means of fluorimetric analysis (FACS, Fluorescence-activated cell sorting) using preferably a cytometer FACSCalibur provided with laser argon 15-mW, 488 nm, air cooled, and collected data re analyzed with a specific software that preferably is the software BD CellQuest. An aliquot of 1 x 10⁴ MSCs of each one cellular suspension at the passage from 3 to 6 that it is intended to be characterized, is at first washed (step 160) with PBS (EuroClone, Milano, Italia) for then being subsequently added (step 161) with bovine fetal serum 161b preferably in concentration equal to 1% (FBS, 10270; Gibco Life Technologies) and then incubated (step 162) for 30 minutes with an antihuman murine monoclonal antibody 162b. The used antibodies all conjugated with ficoeritrine (PE) or fluorescein isothiocyanate (FITC), are the ones that follow: anti-CD146, anti-CD105, anti-CD90 (Serotec, Kidlington, Oxford, UK), anti-CD73, anti-CD29, anti-CD44 (BD PharMingen, San Diego, CA) for surface antigens anti-CD14, anti-CD34, anti-CD45, Human leukocyte anti-antigen (HLA)-DR (Becton Dickinson Biosciences) for the markers of hematopoietic stem cells, and anti-CD80, anti-CD86, anti-CD40 for the co-stimulatory molecules. Antibodies of an associated isotype, conjugated with a fluorochrome and nonreactive are used as antagonists. For each sample, a total of 10000 events is acquired and analyzed (step of analysis of samples 163). All the cellular preparations hall express the typical markers of the MSCs: CD29, CD44, CD90, CD73, CD105, CD146, but not the hematopoietic markers CD45, CD14, CD34 and the co-stimulatory molecules CD80, CD86, CD40, and HLA of class II.

The capacity of differentiation in adipogenic and osteogenic sense are tested by means of a protocol yet described (described in t'Anker PS, et al., Mesenchymal stem cells in human second-trimester bone marrow, liver, lung and spleen exhibit a similar immunophenotype but a heterogeneous multilineage differentiation potential. Haematologica 2003; 88:845-852) that anyway is modified as follows.

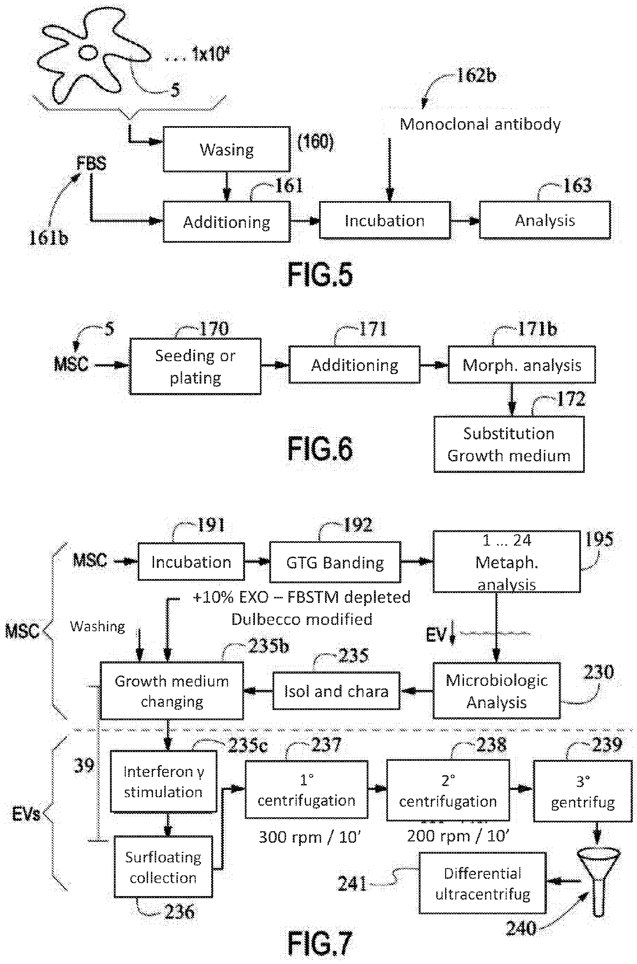

As it is shown in figure 6, the differentiation in adipogenic sense is performed by means of seeding 170 of 2 x 10⁴ cells/cm² in MSCs specific growth medium (Lonza) at 37° C in humidified atmosphere at 5% of CO₂ up to 100% of confluence. For stimulating an optimal adipogenic differentiation, following the seeding 170, the culture are added (step 171) for two weeks in Mesencult medium and the Mesencult adipogenic stimulating supplement (Stem Cell Technologies, Vancouver, BC, Canada), following the instructions of the producer.

The accomplished differentiation is evaluated through morphological analysis (step 171b) of the drops of fat following the coloring with a lyso chromic azo compound like preferably the Oil Red OR (Sigma-Aldrich). For evaluating the osteogenic differentiation, the MSCs are plated to a density of 5 x 10³ cells/cm² and cultivated up to 50% of confluence in medium for MSCs (Lonza) at 37° C in humidified atmosphere at 5% of CO₂.

The induction of a phenotype osteogenic is performed by means of a specific step of substitution of growth medium 172 (performed in parallel to the adding of the culture 171) by substituting the growth medium with an osteogenic specific Medium (Lonza) that is then changed every 3-4 days for 2-3 weeks. The osteoblasts are identified by means of the detection of the calcium deposits with the red alizarin (Sigma-Aldrich) coloring.

Both the processes of differentiation are monitored by combined digital camera with a inverted microscope.

For what attains to the genomic stability analysis, the population of MSCs is therefore subjected to a genomic stability analysis. In this phase of analysis, a sample of 1 x 10⁵ cells form each lot of MSCs is subjected to conventional karyotype analysis using standard techniques, like for example those which are described in Brambati B, Simoni G: Diagnosis of fetal trisomy 21 in the first trimester. Lancet 1983; 1,586. The metaphases are obtained from MSCs cultures after a phase of incubation (step 191) with Colcemid (IrvineScientific) at 1 mg/ml of final concentration for 5 hours at 37° C before their collection. The chromosomes are then subjected to GTG-banding (karyotype aploid of bands 400) 192, and the formula for the karyotype is defined following the recommendations of the Human Cytogenic International System published in 2009. Preferably, from 8 to 24 metaphases are analyzed at every established passage. From the analysis 195 results if the karyotype is such as to allow the preparation of the EVs 10 or less. In detail, potential anomalies found in more than two metaphases in the same passage in culture are considered clonal. The karyotype performed on every lot of MSCs shall result normal, without numeric cytogenic or clonal structure alterations to the end of being used for the preparation of EVs 10.

Then a microbiologic analysis 230 is performed.

The microbiologic tests for anaerobic and aerobic bacteria, endotoxins and mycoplasmas are performed following the standard protocols and shall result negative.

Then, a step of isolation and characterization 235 of the EVs 10 is performed.

The surfloating necessary for the preparation of EVs 10 are obtained from the same lot of MSCs coming from each sample of chorion, at the selected passage, removing (step 235) the growth medium Chang C from the flask and then washing with PBS and substituting the medium with Dulbecco Modified Eagle Medium 1x (DMEM) supplemented with 10% Exo-FBSTM Exosome-depleted (#EXO-FBS-50A-1 Biosciences system; Nord Whisman Rd Mountain View, CA, USA) in substitution of the usual FBS, to the end of avoiding potential contaminations of vesicles deriving from other sources, with recombining human interferon-y (25 ng/mL/day, Sigma-Aldrich) (step 235c) since this treatment has advantageously demonstrated suitable of enhancing the immunologic properties of the MSC.

This inflammatory cytokine is produced during an T-helper 1 immune response and is increased in many autoimmune diseases, among which the multiple sclerosis, diabetes mellitus of first type, rheumatoid arthritis and the same celiac disease. The exposure of the MSCs to the IFN-gamma has demonstrated increasing both their immune-suppressive reactivity, most of all inducing the di-oxygenase indolamine enzyme as well as the immuno-modulatory function through the production of molecules like the Transforming Growth Factor-β and the hepatocytes growth factor. Furthermore, Noone et al. Have demonstrated that the preconditioning with IFN-gamma protects the MSCs from the cellular- mediated death induced by the natural killer cells (Noone C, Kihm A, English K, OR'Dea S, Mahon BP. IFN-y stimulated human umbilical-tissue-derived cells potently suppress NK activation and resist NK-mediated cytotoxicity in vitro. Stem Cells Development 2013; 22: 3003-14.), a mechanism which is considered responsible of the premature disappearance of the MSCs once injected "in vivo" in an allogenic setting.

Concluding there are clear evidences of a powering of the regenerative and anti-inflammatory capacity of the MSCs following of an inflammatory stimulus.

After three days of incubation, the surfloating are therefore collected (step 236), joined back together, and subjected to a first centrifugation 237 at 300g for 10 minutes for removing the cells, and subsequently to a second centrifugation 238 at 2000 g for 10 minutes for eliminating the dead cells, and finally to a third centrifugation 239 at 10.000 g for 30 minutes for eliminating the cellular detritus. Said third centrifugation 239, even if being performed at an high g number, does not represent for the purposes of the present invention a step of ultracentrifugation, in which in contrast the magnitude of acceleration in g is of a upper measure (1x10⁵).

During this procedure, the pellets are thrown away, and the surfloating are using for the subsequent passage. After the last centrifugation, the surfloating is instead used into the filtration (step 240) and subsequent differential ultracentrifugation (step 241).

For what deals with the further phase of ultracentrifugation 250, Théry et al. in the their document "Isolation and characterization of exosomes from cell culture supernatants and biological fluids", Curr Prot Cell Biol 2006; suppl:3.22.1-3.22.29, have provided a detailed description of the procedure which is mostly applied for isolating and characterizing the exosomes from the cellular surfloating to which the procedure of ultracentrifugation 250 object of the present invention takes inspiration, respective to which some adaptations are brought.

For sterilizing the preparation and removing the greater vesicles (the apoptotic bodies), the surfloatings are at first subjected to a procedure of filtering 251 (that corresponds to 240 in the previous figure) through membranes with pores of the diameter of 0,22 mm.

Subsequently, aliquots of 10 ml are subjected to a first ultracentrifugation 252 at 100.000 g (preferably, by means of the use of a Optima L-90K; Beckman Coulter ultra centrifuge) for 1 hour at 4° C, then the pellets are washed (step of washing 252b) in a serum-free medium (PBS, Sigma) and subjected to a second ultracentrifugation 253 at the same conditions to the end of eliminating the contaminating proteins.

The final pellets 9 containing the EVs 10 are newly suspended (step 254) in a physiologic solution of sodium chloride at 0.9% containing preferably 10% of dimetyl sulphoxilde (DMSO, WAK-Chemie Medical GmbH, Steinbach / Ts, Germania) and preferably 5% of human albumin (Baxter, Roma, Italia), at a final concentration of 1 unit of EVs 10 per 1 ml, and preserved as aliquots of 1 ml at -80° C up to their use obtaining an intermediate preparation 900 ready for being subjected to a further procedure of control, and that becomes the "preparation" properly said of the present invention only following a further procedure of washing and new suspension.

To this end, the fraction of EVs 10 prepared from the surfloating of 1 x 10⁶ MSCs is defined as 1 unit. The contamination from endotoxins of every preparation of Evs 10 is excluded by means of the test of Limulus according to the instructions of the producer (Charles River Laboratories, Inc.; Wilmington, MA, USA). It is underlined that, due to the lack of methods being more accurate than the present for obtaining a physical and punctual separation of the smaller micro vesicles from exosomes, it cannot be excluded that the preparations of EVs 10 could also contain minimal proportions of micro vesicles. Finally, the number of EVs 10 typically obtained varies from 1.600 to 4.000 particles/cell, with a mean value of 2.400 particles/cell (corresponding to 2,4 x 10⁹ particles/ml of surfloating).

For what concerns the morphological aspect of the preparation to the end of valuating if the so obtained preparation is actually constituted by purified EVs 10 or by contaminating material, it is performed an analysis at the electron microscope as follows: a pellet 9 (residual part at the bottom of the cuvette 9c) of EVs 10 is newly suspended (step 260) in 100 ml of paraformaldehyde at 2%, then 5 ml of this mixture are introduced (step 261) in a Formvar-carbon grill for the electronic microscope, they are covered and left for 20 minutes in a dry atmosphere for allowing the adsorption of the membrane. After, drops of PBS of a 100 microliters volume are made fall (step 262) on a polyolefin and paraffinic wax-based material as the Parafilm where grills are transferred by using a clean jaw having the attention of keeping the side of the membranes downwards. After having added a drop of glutaraldehyde 264g with a volume of 50ml in concentration preferably equal to 1% for 5 minutes, the grills (grids) are washed (step 263) preferably eight times through passages in 100 ml of distilled water for 2 minutes each. Then the samples are contrasted (step 264) with a drop of 50 µl of uranyl oxalate solution 264u with pH 7,0 for 5 minutes, and then they are incorporated in a mixture of uranyl acetate at 4% (step of incorporation in uranyl mixture 264i) and 2% methylcellulose in a ratio of 100µl/900µl respectively, for 10 minutes in ice and removed (step 265) with inox steel pliers, having the attention of eliminating the excess of liquids by means of the use of filter card. Finally, the grills are dried (step 266) at the air for a time variable from 5 to 10 minutes, up to which a blue-gold color develops. The grills can be preserved in special collectors for many years, and examined with an electron microscope of the type Jeol JEM 1010 (JEOL Inc.; USA) a 80 kV. The morphological analysis shall show EVs 10 with spheroidal morphology and of size comprised between 50 and 160 nm.

The EVs 10 are too small for being directly analyzed at the cytofluorimeter. For overcoming this problem, the EVs 10 are fixed to microspheres or balls (beads) having a size such to being detectable with a flow cytofluorimeter. For said scope, the EVs 10 are made adhere (step 267) to specific balls of sulphated latex (4 µm; Molecular Probes, Invitrogen) for 30 minutes at ambient temperature, then washed in 0,5% of bovine albumin serum and newly suspended in a MES buffer (0,025 M MES, 0.154M NaCl, pH 6,0). The EVs 10 (10 mcg) are then incubated for 30 minutes at ambient temperature in 100 microliters containing the corresponding antibody conjugated with the fluorophore and then diluted in 300 microliters and immediately acquire. For said analysis, the following antibodies are used: FITC- or PE-conjugated against CD34, CD44, CD45, CD73, CD105, CD90, CD146 (Becton Dickinson Biosciences). The respective isotypical IgGs FITC or PE of rat, non immunes (Miltenyi Biotec, Bergisch Gladbach, Germany) are used negative antagonists. For each sample, a total of 10.000 events is acquired and analyzed.

A sample of EV 10 of every lot is therefore subjected to analysis of the protein content.

For determining the protein content, the kit of analysis of the proteins Bradford (BioRad, Hercules, CA, USA) is employed on 100 mg (10 ml of every sample) of the preparation of EVs 10. The measurement of the total amount of proteins which are present in the preparations of EVs 10 provides for an approximate estimation of the efficiency of the MSCs into the production thereof.

The composition so obtained is used therefore for therapeutic use in celiac patients, with disease being active as well as treated, that undergo both to laboratory tests that to a gastro-esophagus-duodenoscopy with bioptic samples of the duodenal mucosa in the field of the normal iter of exams for their disease.

All the patients shall be diagnosed as being affected by celiac disease following the widely accepted criteria, and are considered "active" if they result positive for the anti-endomysium and anti-transglutaminase of class A antibodies and have the typical mucosal lesions at the histologic exam in diet containing gluten, while they are considered "treated" if they result negative for the anti-endomysium and anti-transglutaminase of class A antibodies and have a critical recovery from the mucosal lesions at the histologic exam following of a gluten-free diet during at least 12 months. The evaluation clinical helps itself also with an objective exam, with the calculation of the body mass index and of routine laboratory exams, with the D-xylose test for establishing the intestinal absorption function, and the Elispot test for detecting potential gluten-reactive T cells T circulating.

The patients are excluded if they have significant co-morbidities like psychiatric diseases, organ insufficiency, concomitant infections or immunodeficiencies, while are included if affected by a concomitant autoimmune disease, like the diabetes mellitus of type 1, autoimmune thyroiditis, autoimmune arthritis, etc since it is plausible that also these conditions joy of the treatment according to the present patent. It is specified that every patient is treated with the same lot of EVs 10 towards which the immunologic reactivity was previously tested, as follows.

For reducing the risk of unattended immunologic reactions to the administration of the preparation of EVs 10, for each patient a test of lymphocyte proliferation "in vitro" is performed before starting the treatment, using a sample of the same lot of EVs 10 that will be used for the therapy and a sample of peripheral blood of the patient from which PBMCs are isolated, by means of centrifugation on a gradient of density (Ficoll-Paque PLUS, Amersham Biosciences, Bucks, UK). Said test represents a test of evaluation "in vitro" of potential adverse immunologic response.

After separation and washing with PBS preferably for two times, the cells are recuperated (step 280) and suspended in a Roswell Park Memorial Institute (RPMI) 1640 (Gibco, Life Technologies Ltd) medium supplemented with 10% of human serum deactivated with heat, added with 100 U/ml of penicillin and 100 □g/ml of streptomycin (all coming from Lonza). The vitality of the cellular suspension is therefore determined in a step of determination of the cellular vitality 290 by means of trypan-blue test of exclusion and the sample is not used if the vitality is lower than 95%. Furthermore, from this cellular population, are also purified the T- lymphocytes by means of immuno-magnetic negative selection by using the kit Pan T II (Miltenyi Biotec) following the instructions provided by the company.

Subsequently, the PBMCs and the T- lymphocytes are once more suspended (step 291) separately in a growth medium (10⁵ cells/ml) for being reused in the sample that follows.

In a plate 295 with 96 holes with rounded bottom (Costar®, Corning, NY, USA) are disseminated 3x10⁴ T- lymphocytes for each hole in triplicate, in presence of 1x10⁵ autologous PBMCs previously irradiated (3500 rad) and used as cells having the antigen, and an unity of EVs 10 used pure and diluted 1:2 and 1:10, in a final volume of 250 µl of RPMI 1640 (Gibco). As controls, the lymphocytes are incubated (step 292) in absence of EVs 10 (medium alone) or stimulated with the phytohaemagglutinin polyclonal mitogen (1 µg/ml; Boehringer, Mannheim, Germany). After an incubation of 48 hours at 37° C in humidified atmosphere of CO₂ al 5%, the surfloatings are collected and freezed (step 293) a -20° C for the subsequent determination of the levels of IFN-gamma using a kit ELISA (Pierce Endogen, Rockford, THE, USA), while the T cells are pulsed with [³H]thymidine (0,5 µCi/pit; GE Healthcare; Buc Cedex, France) during the night, and then collected for the evaluation of [³H]-thymidine by means of a liquid scintillation counter and the results are expressed as counts per minute (cpm). The cells are considered immunologically reactive if the levels of IFN-gamma [indicated as fold increase (FI), that is the level of IFN-gamma in culture with EV/level of IFN-gamma in culture with only means], and/or the cellular proliferation [indicated as stimulation index (SI), that is, the value of cpm in culture with EV/cpm in culture with only the medium] result ≥2. If those preliminary experiments do not evidence any reaction, the infusion "in vivo" is performed.

The treatment of the patient takes place with the procedure hereinafter described: in the morning of the established day, the unit of EVs 10 necessary for treating the single patient are thawed and newly suspended in a vehicle constituted by physiologic solution of sodium chloride (0,9%) and human albumin at 20% (3/1 v/v) to a density of 1 unit of EVs 10 in 1 ml of solution in a sterile syringe that is immediately (within 30 minutes) delivered to the bed of the patient. For reducing the risk of potential collateral effects, classified according to established categories in the medical dictionary for regulating the systemic activity, only a tenth of the dose of the preparation of EVs 10 estimated as being necessary is initially intravenously administered.

If within two hours there are no collateral effects, the total of the preparation of EVs 10 (corresponding to a 1 unit/pro-kg of body weight) is administered by means of slow intravenous infusion during 1 hour. Finally, for reducing the risk of allergic reaction, a premedication with anti-histamine drug (Clorfenamine 10 mg intramuscular) is performed before the first infusion.

After each infusion, the patient is made to rest in bed for 4 hours, during which the vital parameters are monitored as well as the potential adverse reactions. Since the duration of the effectiveness of said treatment cannot be foreseen and the detailed mechanisms thereof are not known, it is foreseen that the infusion is repeated every 3 months.

The evaluation of the safety of said treatment includes the analysis of the system ic tolerance, of potential collateral effects and of serious adverse events (that is risk of life, or condition requiring hospitalization, or to which follows invalidity or death). The serious adverse effects are registered at the moment wherein they take place, while the collateral effects are registered every 6 months from the practitioner which is responsible of the treatment. Furthermore, the monitoring of unpredicted events is provided, with particular regard to any opportunistic infection or to the appearance of signs or symptoms of tumor.

The effectiveness of the therapy is defined as the disappearance or missed re-appearance of the serologic and histologic indexes of disease, that is the anti-endomysium and anti-transglutaminase of class A antibodies and the typical lesions of the mucosa which are morphometrically valuated in patients with active or treated disease, respectively, and a diet containing gluten. During the control visits, programmed every three months, it takes place the evaluation of the general conditions of the patient, by monitoring the gastrointestinal symptoms, registering the potential adverse effects, performing potential laboratory tests, and the IFN-y Elispot sampling to the end of testing the reactivity to gluten of circulating T-lymphocytes that could anticipate the development of intestinal lesions, while the endoscopic exam with duodenal biopsy is repeated after 12 months.

If the clinical and laboratory frame show a state of quiescence of the disease, the infusion of EVs 10 is performed. Subsequently, the follow-up will be performed by means of the plasmatic D-xylose test annually made for valuating the intestinal absorption capacity, therefore avoiding the endoscopy that represents an invasive exam.

For having a measurement of the damage of the intestinal mucosa (mucosal morphometry) and following the adaptations in time thereof, it will be used the morphometric analysis using the calculation of the radio surface/volume ratio performed on duodenal samples included in paraffin and fixed in formalin, using a Weibel reticulum (Tunbridge Wells, England, UK) inserted in the microscope eyepiece, according to the method of Dunnill and Whitehead, as standardized by Corazza et al. A value lower than a 30 is considered indicative of atrophic mucosa. Furthermore the number of intra-epithelial lymphocytes will be evaluated by means of immunohistochemical technique on serial colored sections with the polyclonal rabbit antibody anti-CD3 human (#A 0452; Dako, Denmark), following the instructions presented on the datasheets. The differential counts for each field will be performed by an expert hystopatologon by means of an optic microscope (ZEISS Axioplan 2, Oberkochen, Germany and the results expressed as percentage of epithelial cells. With the test Interferon-y Elispot it is intended to detect the presence of circulating gliadin-T specific lymphocytes following a recently developed method that bases on the proof that the ingestion of gluten in celiac patients causes the expansion of the cells T of the memory gluten-reactive detectable by means of IFN-y enzyme-linked immunospot (ELISPOT).

In detail, in a first phase 550 a sample of peripheral blood (15-20 ml) is collected in an heparinized syringe 550s and the PBMC are subjected to a phase of insulation 555 by means of centrifugation (step of centrifugation of the peripheral blood 555c) over a gradient of density of Ficoll-Paque and then washed in PBS in a subsequent step of washing 555e. Aliquots of 4 x 10⁵ cells for each hole are seeded in duplicates in plates with 96 holes (Millipore, Bedford, MA, USA) covered with human purified antibodies anti-IFN-gamma 556 (MabTech; Nacka Strand, Sweden) in 200 ml of medium complete X-VIVO15 integrated with human serum 557 in concentration preferably equivalent to 5% deactivated by heat (Lonza), 1% of antibiotic 558 (100 U/ml of penicillin and 100 µg/ml of streptomicine) and 1% of L-glutammine (2 mM) (all being provided by BioWhittaker, Verviers, Belgium).

The antigenic preparation of gliadin (see below - 559) is used in a concentration of 50 mg/ml. The antigenic preparation of gliadin serves as a base for for the subsequent test of positive control being performed using the phytohaemagglutinin. In fact, in a subsequent phase of control 560, the phytohaemagglutinin (Sigma) is used as a positive control to a final concentration of 2,5 mg/ml, while the complete growth medium is used as a negative control. The cells are then incubated for 36 hours (step of incubation 561; first phase of incubation of the cells) with the human biotinylated antibodies anti-IFN-y (MabTech), to which following the incubation with streptavidin perossidase (BD-Pharmingen, San Diego, CA, USA) (step 562, second phase of incubation). The spot forming cells (SFC) are finally counted (step of counting 563) in each hole using an automated immunospot reader (preferably, but not exclusively A.EL.VIS, Hannover, Germany), and the results expressed as IFN-γ-SFC/4 × 10⁵ PBMC (SFC detected in presence of gliadin subtracted the SFCs detected with the growth medium). The patients are considered gluten-sensitive if they show an increment of SFC in response to the gliadin in ate lest three times the value observed with the negative control (≥3 times).

The peptic-tryptic digest of gliadin is prepared as briefly described hereinafter. In brief the gliadin of wheat (100 mg; Sigma-Aldrich) is diluted in HCl 0,1N (10 mL) is incubated at first with 500 mg of pepsin (Sigma) for 2 hours at 37° C, and then with 500 mg of trypsin (Sigma) at pH 7,8 for further 2 hours. The proteic concentration of the gliadin hydrolyzed is determined with the method BioRad Protein Assay Dye (# 500-0006; BioRad). Said digest is then subjected to a de-starching by means of the tissue transglutaminase enzyme (T5398, Sigma) for 4 hours in 0,125 mol/L Tris-HCI, pH 8,5 (ratio TG2/antigen: 1/10), as already standardized.

Finally, the aliquot of said antigenic preparation are dried and preserved at -80°C. Said preparation is indicated with the wording "gliadin" in the course of description of the present patent application.

The advantages of the composition object of the present invention are clear in view of the aforementioned considerations and of the description. In detail the applicant has already shown that the MSCs from marrow exert a powerful anti-inflammatory action "in vitro" on the gliadin-T specific lymphocyte of patients affected by celiac disease both in terms of suppression of the antigen-specific reactivity as well as by means of the induction of a tolerogenic pattern (Ciccocioppo R, Camarca A, Cangemi GC, Radano G, Vitale S, Betti E, Ferrari D, Visai L, Strada E, Badulli C, Locatelli F, Klersy C, Gianfrani C, Corazza GR. Tolerogenic effect of mesenchymal stromal cells on gliadin-specific T lymphocytes in celiac disease. Cytotherapy; 2014: 16: 1080-91). The bioactive molecules which are responsible of said effects are all contained in the extracellular vesicles that are preferred to MSCs induced for the therapeutic clinical use since advantageously produced, preserved and capable of reaching the target organ (intestine) place of the damage.

Furthermore, from a clinical study performed by the applicant, it has been demonstrated the feasibility, safety and effectiveness of serial intravenous infusions of autologous MSCs of marrow into the refractory celiac disease (RCD) of type II, as it is defined by the presence of a severe syndrome of malabsorption with atrophy of the intestinal mucosa, no more being responsive to a gluten-free diet and with an high percentage of aberrating intra-epitelial lymphocytes. Said condition does not have a safe and effective standardized therapy and is worsened by a negative prognosis due to the high risk of developing intestinal lymphoma. According to these premises, a 51-years old patient affected by RCD of type II and refractory to the steroid therapy, has been subjected to said treatment.

Of the said patient, have been monitored: malabsorption indexes, architecture of the mucosa, percentage of intra-epithelial aberrating lymphocytes, both at the moment of the enrollment and during each infusion, as well as after 6 months from the treatment.

It has been furthermore determined the mucosa expression of the interleukin (THE)-15 and of its receptor, that play a crucial role into the pathogenesis of this condition, and, more in general, in the damage of the intestinal mucosa into the celiac disease.

By the results of the study, following of the verification of the feasibility of the MSCs expansion and of a series of four systemic infusions of 2x10⁶ MSC/kg every four months for one year, without adverse effects, it has resulted that the patient has shown a gradual and long-lasting enhancing of his condition, with a normalization of the evacuation frequency, of the body mass index and of the laboratory test of the mucosal architecture.

The following table shows the track of the clinical features of the patient.

| Parameter | Before the 1° infusion of MSC | At the 2° infusion of MSC | At the 3° infusion of MSC | At the 4° infusion of MSC | Six months after the last infusion |
|---|---|---|---|---|---|
| Body mass index | 14.9 | 16.2 | 15.4 | 19.0 | 18.6 |
| Xylosaemia | 12.2 | 17.6 | 14.2 | 42.7 | 38.9 |
| Haemoglobulin Red cells | 13.2 | 11.6 | 12.2 | 12.1 | 13.4 |
| | 4.34 | 3.8 | 4.21 | 4.18 | 5.29 |
| White cells | 14.51 | 8.30 | 11.6 | 8.12 | 9.10 |
| N/L/M/E/B | 74/20/4.4/0.9/0.7 | 49/36/12/2.3/0.7 | 69/18/9/2.3/1.7 | 49/33/12/4.8/1.2 | 49/3 4/12/ 3/1.0 |
| Platelets | 364 | 378 | 396 | 414 | 441 |
| Albumin | 1.9 | 2.5 | 3.4 | 3.9 | 3.4 |
| γ - globulin | 26.8-2.0 | 31.6-1.6 | 28.7-2.2 | 24.7-1.8 | 23.6-1.8 |
| IgG-A-M (mg/dl) | 1490/504/37 | 1660/591/58 | 1780/572/45 | 1700/524/46 | 1640 /596/ 51 |
| Na/K/Cl | 142/1.9/109 | 140/2.5/111 | 143/2.6/113 | 143/3.5/112 | 141/ 3.78/ 108 |

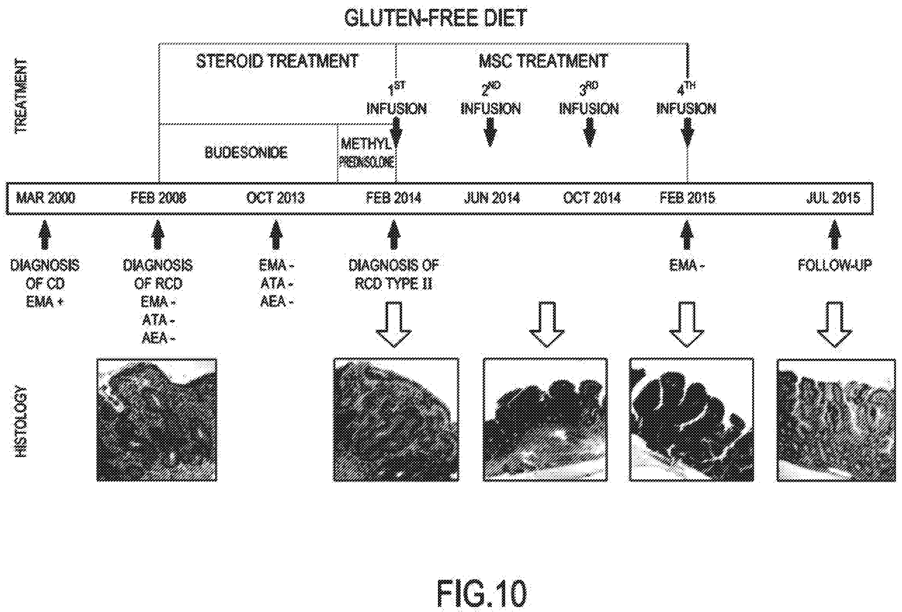

Figure 10 shows a mixed diagram relating to the therapeutic scheme of the patient with MSCs following the failed response to the treatment with steroids. From the diagram of figure 10 it is observed a significant enhancement of the mucosal architecture, up to a complete normalization.

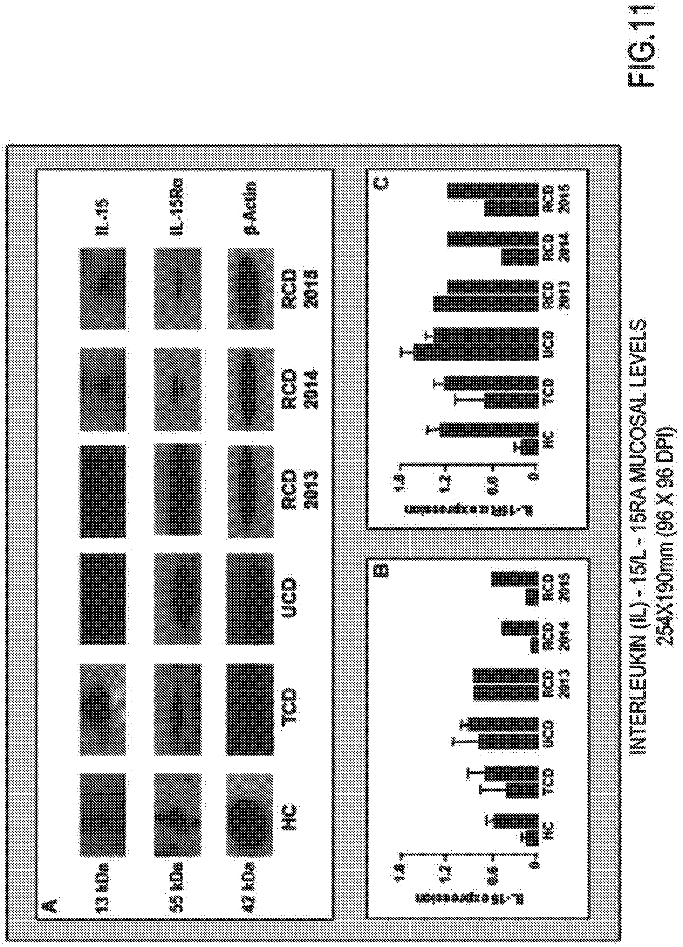

In detail, the expression of THE-15 and of its receptor had undergone a significant decrease up to a substantial disappearance at the end of the cycle of therapy with MSC (figure 11). Therefore, the treatment of the RCD of type II with serial infusions of autologous MSC from marrow has been shown promising, bringing to a complete recuperation from a condition potentially with negative prognosis, blocking the harmful effects linked to the hyper production of (THE)-15. Said case in in course of publication (Ciccocioppo R, Gallia A, Avanzini MA, Betti E, Picone C, Vanoli A, Paganini C, Biagi F, Maccario R, Corazza GR. A refractory celiac patient successfully treated with mesenchymal stem cell infusions. Mayo Clinic Proceeding 2016, June)

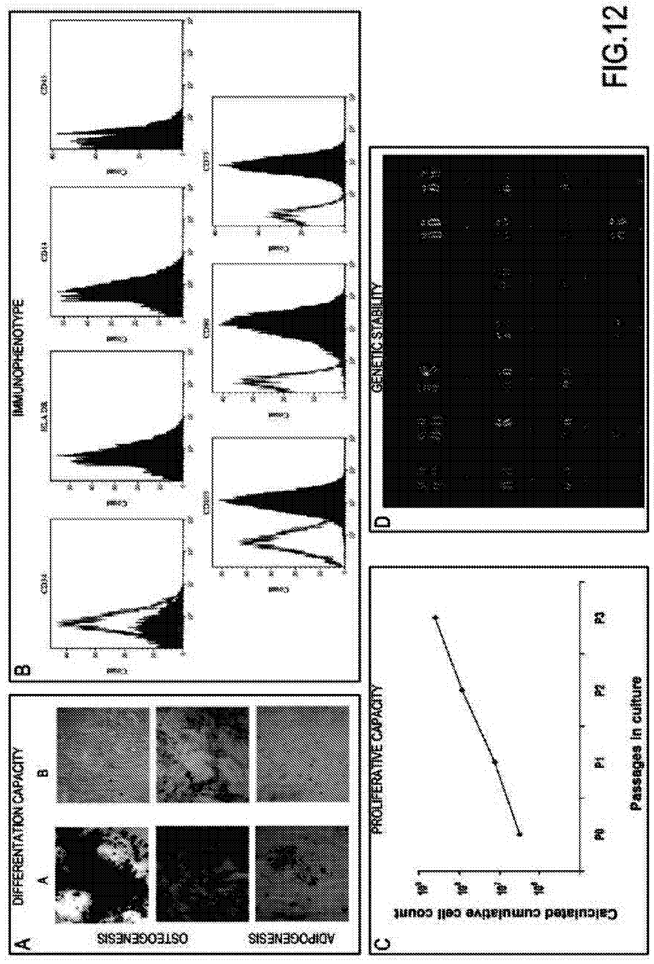

Figure 12 shows the characterization of the mesenchymal cells from marrow of the patient therein described. In the figure there are three diagrams:
A) Relating to the capacity of osteogenic and adipogenic differentiation, shown by the histologic individuation of calcium deposits positive to the Red Alizarine (or Mordant Red) and of the activity of the alkaline phosphatase that shows a differentiation in the osteoblasts, and lipids which are OR-positive to the red oil of the adipocytes. The cells of negative control are represented in the frames on the right.
B) Immunophenotype characterization through the flow cytometry, indicating the positivity to antigens CD 105, CD 90, CD 73, and the negativity for the CD34, CD14, CD45 and the molecules of histo-compatibility (HLA)-DR.
C) There is reported a proliferative capacity calculated as the sum of the cumulative counting of cells from passage (P)0 al P3.
D) Cytogenic analysis through the conventional karyotype, that shows the absence of aberrating chromosomes.

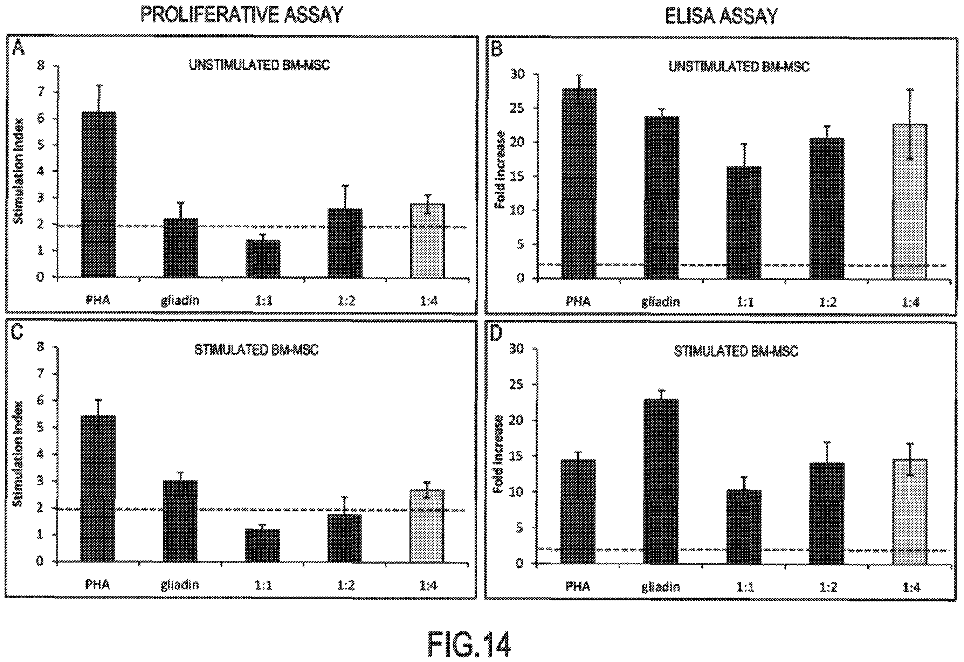

Figure 14 shows the curve of growth of mesenchymal cells which are isolated from placenta's chorion of three different donors, with logarithmic ordinate in accordance to the days passed in culture and the duplication time. It is observed a systematic increment of the cells and of their duplication.

From said samples of chorial MSC (C-MSC) and from MSC previously isolated from the marrow blood (BM-MSC) of three healthy donors, the Evs have been isolated. Furthermore in detail, the EV have been obtained both from non-stimulated MSC, and from MSCs stimulated with interferon-y (25 ng/mL/per day, for a number of days variable between 3 to 7). To the end of valuating the immuno-suppressive effect of said EV on the antigen-specific adaptive response of the celiac disease, the gliadin-T specific lymphocytes isolated from the mucosa of the affected patients, have been incubated in absence (ctr-TLCs) and in presence of said EV coming from marrow (BM-EV) and from chorion (C-EV) (Interferon), at three different ratios (EV:T-cell 1:1, 1:2, 1:4). The effect of the EV has been evaluated in terms of mortality of the T- lymphocytes, production of interferon-y (expressed as Fold Increase) by means of ELISA test and proliferative response (expressed as Stimulation Index) following of gliadin re-stimulation.

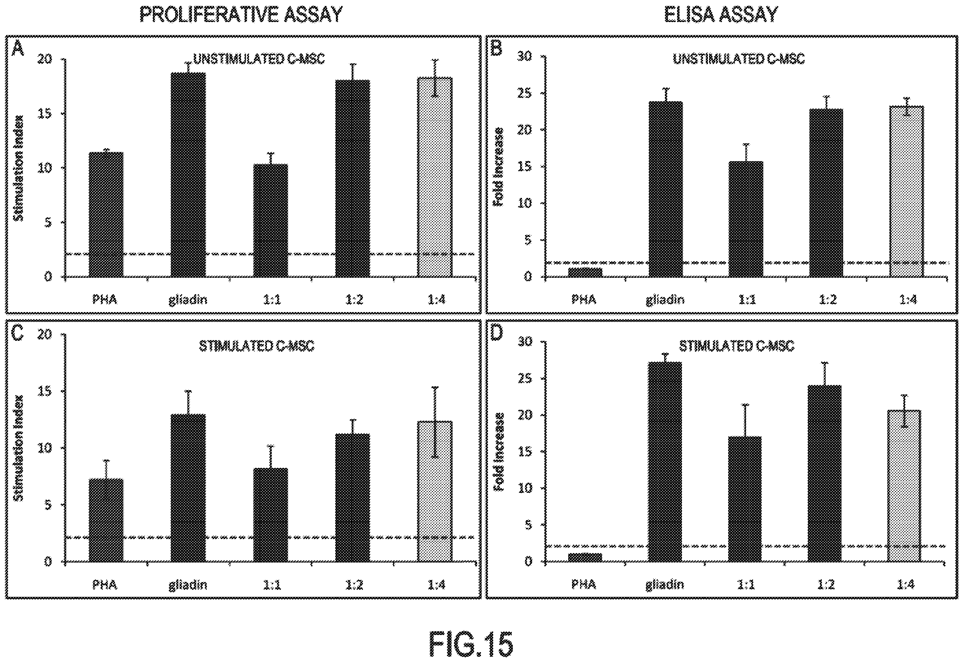

The results of said experiments (shown in figure 15 for what concerns the BM-EV and in figure 16 for what concerns the C-EV) have first of all demonstrated a marked reduction, invariably >30%, of the number of the vital T- lymphocytes following of incubation with C-EV and BM-EV to the ratios 1:1 and 1:2, while it has not been observed a substantial adaptation in the ctr-TCLs and in the cultures with EV-TCL ratio of 1:4. In parallel, it has been observed an increment of the mortality of the gliadin specific T- lymphocytes, as calculated by means of cytofluorimetric analysis, in presence of C-EV and BM-EV, with a gradient which is function of the used ratio (mean values for ctr-TCL= 15% - for BM-EV 1:1=34%, 1:2=25%, 1:4=26% - for C-EV 1:1=38%, 1:2=31%, 1:4=25%). Finally, we have observed an inhibitory effect over the interferon-y production by the BM-EV and by C-EV, most of all using the ratio 1:1 and EVS coming from previously stimulated MSCs. No substantial differences have been observed between the effects of the BM-EV and those of the C-EV, Therefore, these experiments, represent the biologic base to the usage of C-EV obtained from C-MSC stimulated into the therapy of the non-complicated celiac disease, as for the present patent application.

## Claims

1. Method of preparation of a composition comprising extracellular vesicles (10) for treating celiac disease, said method being **characterized in that** it comprises:
- a step of extraction of extracellular vesicles(10) from mesenchymal stem cells (MSC) (5), derived from a single sample of chorion (C) in advance separated from the placenta (200) in a step of separation chorion-placenta (93), and
- a step (251, 252 253) of sterilization and elimination of contaminating proteins from a semi-finished fluid containing said extracellular vesicles (10), wherein said step (251, 252, 253) of sterilization and elimination of contaminating proteins comprises at least a first step of ultracentrifugation (252) of the said semi-finished fluid, and wherein
- said first step of ultracentrifugation (252) is performed following of a phase of filtration (251) of said semi-finished fluid;
wherein said plurality of steps of centrifugation (237-239) takes place on a surfloating portion of a further semi-finished comprising said mesenchymal stem cells (5), said further semi-finished being in advance incubated for a multi-day period of time and being subjected to a treatment of increase of immunologic properties of said mesenchymal stem cells (5), said treatment of increase of immunologic properties of said mesenchymal stem cells (5) comprises a step of washing by means of PBS followed by incubation with human recombinant interferon-y.

2. Method of preparation of a composition according to claim 1, **characterized in that** it comprises a second step of ultracentrifugation (253) of the said semi-finished fluid, wherein said second step of ultracentrifugation (253) is performed after said first step of ultracentrifugation (252) following of an intermediate step of washing (252b), performed between said first and said second step of ultracentrifugation (252, 253) and being performed in a growth medium without serum.

3. Method of preparation of a composition according to claim 1 or 2, **characterized in that** it comprises a plurality of steps of centrifugation (237-239), being performed in advance respective to said first and/or second step of ultracentrifugation (252, 253), wherein said plurality of steps of centrifugation (237-239) separates cellular detritus and/or dead cells from said extracellular vesicles(10), and wherein each of said steps of centrifugation (237-239) is performed for a time greater or equal to 10 minutes, and wherein said semi-finished fluid comprises surfloating elements following the said plurality of steps of centrifugation.

4. Method according to claim 1, wherein the surfloating necessary for the preparation of extracellular vesicles(10), that represent said further semi-finished, are subjected to a step of changing of a growth medium from a first type Chang C to a second type Dulbecco Modified Eagle Medium, and wherein said second type of growth medium is supplemented with a predefined percentage of Exo-FBSTM Depleted Exosome.

5. Method according to any of the previous claims, wherein a plurality of samples of said chorion (C) is subjected to a step of digestion (141) performed in two times, at first by means of the use of pronase and subsequently of collagenase, in percentages preferably and respectively of 0,125 g/L and 0,1g/L.

6. Method according to claim 5, wherein said step of digestion (141) performed in two times is blocked (142) by means of an addition of a balanced saline solution and subsequently through a step of centrifugation (143); and wherein following of the block of the said second time of the step of digestion each pellet produced from said step of digestion is further suspended in a growth medium Chang Medium C supplemented with bovine fetal serum deactivated by heat and with antibiotic for then undergoing to a subsequent step of incubation in humidified atmosphere.

7. Method according to claim 6, wherein following of the said step of incubation in humidified atmosphere, the growth medium is substituted and is performed a step of enzymatic digestion after having removed (147) the growth medium and having added trypsin.

8. Method according to claim 7, wherein said step of digestion is blocked (150) by means of the addition of a predefined percentage of HBSS and by means of a centrifugation (151) following which the pellet is suspended in a growth medium and a control of the vitality of the cells is performed.

9. Method according to any of the preceding claims, comprising a step of analysis of genomic stability (190) wherein a sample of each lot of MSCs is subjected to an analysis of karyotype comprising a step of incubation (191) performed in advance with respect to a step of banding (192).

10. Method according to any of the preceding claims, comprising a step of microbiologic testing for verifying the absence of anaerobic and aerobic bacteria, endotoxins and micoplasmas.

11. Method according to any of the preceding claims, wherein said extracellular vesicles(10) obtained following ultracentrifugation, are made adhere to balls (beads) or spheres of sulphated latex and subsequently subjected to a cytofluorimetric analysis.

12. Method according to any of the preceding claims, comprising a step of evaluation of the composition of the said preparation performed by means of a first step of suspending (260) a pellet (9) in paraformaldehyde followed by a subsequent step of resting in dry atmosphere for allowing the absorption of the membrane, said step of resting being furthermore followed by a step of contrasting (264) the samples with uranyl oxalate and subsequent incorporation of said samples in a mixture of uranyl acetate and methylcellulose.

13. Method according to any of the preceding claims, comprising a step of analysis "in vitro" of the immunologic response to said EV, comprising a step of collection of samples of EV of a same lot that are incubated together with mono-nucleated cells from peripheral blood of a patient affected by celiac disease, obtained by means of centrifugation, recuperation (280) and subsequent suspension in growth medium added with deactivated human serum and with antibiotic preferably comprising penicillin and/or streptomycin, and wherein the cellular vitality is determined by means of a test of exclusion with trypan blue.

14. Method according to claim 13, comprising furthermore a subsequent step of isolation and seeding of T- lymphocytes in a plurality of retention means within which are contained PBMC in advance irradiated and used as cells having antigen, and a step of subsequent addition of said EV within said retention means; said step of addition is furthermore followed by a step of incubation (292) of said lymphocytes or by a step of stimulation with polyclonal mitogen phytohaemagglutinin; said method comprising therefore a step of incubation and of subsequent collection and freezing (293) of the surfloating collected from said retention means for a subsequent determination of level of interferon-y, and comprising furthermore a step of pulsation of the cells T with tritiated thymidine (³H thymidine).

15. Injectable preparation for use in treatment of the celiac disease, **characterized in that** of being realized by means of extracellular vesicles(10) through a process of realization according to any of the claims 1-14.

## Patentansprüche

1. Verfahren zur Herstellung einer Zusammensetzung, umfassend extrazelluläre Vesikel (10), zur Behandlung von Zöliakie, wobei das Verfahren **dadurch gekennzeichnet ist, dass** es umfasst:
- einen Schritt zur Extraktion extrazellulärer Vesikel (10) aus mesenchymalen Stammzellen (MSC) (5), die aus einer einzelnen Probe von Chorion (C) stammen, im Voraus von der Plazenta (200) in einem Schritt des Trennens von Chorion-Plazenta (93) abgetrennt und
- einen Schritt (251, 252, 253) der Sterilisierung und Eliminierung kontaminierender Proteine aus einer halbfertigen Flüssigkeit, die die extrazellulären Vesikel enthält (10), wobei der Schritt (251, 252, 253) der Sterilisierung und Eliminierung kontaminierender Proteine mindestens einen ersten Schritt der Ultrazentrifugation (252) des halbfertigen Fluids umfasst und wobei
- der erste Schritt der Ultrazentrifugation (252) nach einer Filtrationsphase (251) des halbfertigen Fluids durchgeführt wird;
wobei die Vielzahl der Schritte der Zentrifugation (237-239) an einem aufschwimmenden Teil eines weiteren Halbfertigen stattfindet, das die mesenchymalen Stammzellen (5) umfasst, wobei das weitere Halbfertige vorab für einen mehrtägigen Zeitraum inkubiert wird und einer Behandlung zur Erhöhung der immunologischen Eigenschaften der mesenchymalen Stammzellen (5) unterzogen wird, wobei die Behandlung zur Erhöhung der immunologischen Eigenschaften der mesenchymalen Stammzellen einen Waschschritt mittels PBS, gefolgt von Inkubation mit humanem, rekombinantem Interferon-y umfasst.

2. Verfahren zur Herstellung einer Zusammensetzung nach Anspruch 1, **dadurch gekennzeichnet, dass** es einen zweiten Schritt der Ultrazentrifugation (253) des halbfertigen Fluids umfasst, wobei der zweite Schritt der Ultrazentrifugation (253) nach dem ersten Schritt der Ultrazentrifugation (252) im Anschluss an einen Zwischenwaschschritt (252b) durchgeführt wird, der zwischen dem ersten und dem zweiten Ultrazentrifugationsschritt (252, 253) durchgeführt wird und in einem Wachstumsmedium ohne Serum durchgeführt wird.

3. Verfahren zur Herstellung einer Zusammensetzung nach Anspruch 1 oder 2, **dadurch gekennzeichnet, dass** es eine Vielzahl von Zentrifugationsschritten (237-239) umfasst, die vorab bezogen auf den ersten und/oder zweiten Ultrazentrifugationsschritt (252, 253) durchgeführt werden, wobei die Vielzahl von Zentrifugationsschritten (237-239) Zelltrümmer und/oder tote Zellen von den extrazellulären Vesikeln (10) abtrennt und wobei jeder der Zentrifugationsschritte (237-239) für eine Zeit durchgeführt wird, die größer oder gleich 10 Minuten ist, und wobei das halbfertige Fluid aufschwimmende Elemente aufweist, den mehreren Zentrifugationsschritten folgend.

4. Verfahren nach Anspruch 1, wobei das zur Herstellung von extrazellulären Vesikeln (10), die das weitere Halbfertige darstellen, erforderliche Aufschwimmende einem Schritt des Wechsels eines Wachstumsmediums von einem ersten Chang-C-Typ zu einem zweiten Dulbecco-Modified-Eagle-Medium-Typ unterzogen wird, und wobei der zweite Typ von Wachstumsmedium mit einem vordefinierten Prozentsatz an Exo-FBSTM-Depleted-Exosom ergänzt ist.

5. Verfahren nach einem der vorhergehenden Ansprüche, wobei eine Vielzahl von Proben des Chorion (C) einem Verdauungsschritt (141) unterworfen wird, der zweimal durchgeführt wird, zunächst unter Verwendung von Pronase und anschließend von Kollagenase, in Prozentsätzen bevorzugt und jeweils von 0,125 g/L und 0,1 g/L.

6. Verfahren nach Anspruch 5, wobei der zweimal durchgeführte Verdauungsschritt (141) durch Zugabe einer ausgeglichenen Salzlösung und anschließend durch einen Zentrifugationsschritt (143) blockiert wird (142); und wobei nach dem Blockieren des zweiten Verdauungsschritts jedes Pellet, das durch den Verdauungsschritt hergestellt wurde, weiter in einem Wachstumsmedium Chang-Medium-C suspendiert wird, das mit durch Hitze deaktiviertem fötalem Rinderserum und mit Antibiotikum ergänzt ist, um es dann einem anschließenden Inkubationsschritt in angefeuchteter Atmosphäre zu unterziehen.

7. Verfahren nach Anspruch 6, wobei nach dem Schritt der Inkubation in einer angefeuchteten Atmosphäre das Wachstumsmedium substituiert wird und ein enzymatischer Verdauungsschritt durchgeführt wird, nachdem das Wachstumsmedium entfernt (147) und Trypsin zugesetzt wurde.

8. Verfahren nach Anspruch 7, wobei der Schritt des Verdauens mittels der Zugabe eines vordefinierten Prozentsatzes von HBSS und mittels einer Zentrifugation (151) blockiert wird (150), wonach das Pellet in einem Wachstumsmedium suspendiert wird und eine Kontrolle der Vitalität der Zellen durchgeführt wird.

9. Verfahren nach einem der vorhergehenden Ansprüche, umfassend einen Analyseschritt der genomischen Stabilität (190), wobei eine Probe jeder Charge von MSCs einer Analyse des Karyotyps unterzogen wird, die einen Inkubationsschritt (191) umfasst, der vorab in Bezug auf einen Schritt des Bandings (192) durchgeführt wird.

10. Verfahren nach einem der vorhergehenden Ansprüche, umfassend einen mikrobiologischen Testschritt zum Nachweis der Abwesenheit von anaeroben und aeroben Bakterien, Endotoxinen und Mikroplasmen.

11. Verfahren nach einem der vorhergehenden Ansprüche, wobei die nach der Ultrazentrifugation erhaltenen extrazellulären Vesikel (10) an Kugeln (beads) oder Sphären aus sulfatiertem Latex haften gelassen und anschließend einer cytofluorimetrischen Analyse unterzogen werden.

12. Verfahren nach einem der vorhergehenden Ansprüche, umfassend einen Bewertungsschritt der Zusammensetzung der Zubereitung, der mittels eines ersten Schritts des Suspendierens (260) eines Pellets (9) in Paraformaldehyd durchgeführt wird, gefolgt von einem anschließenden Schritt des Ruhens in trockener Atmosphäre, um die Absorption der Membran zu ermöglichen, wobei dem Schritt des Ruhens ferner ein Schritt des Kontrastierens (264) der Proben mit Uranyloxalat und anschließender Einarbeitung der Proben in ein Gemisch aus Uranylacetat und Methylcellulose folgt.

13. Verfahren nach einem der vorhergehenden Ansprüche, umfassend einen Schritt der Analyse "in vitro" der immunologischen Antwort auf das EV, umfassend einen Schritt des Sammelns von EV-Proben einer selben Charge, die zusammen mit mononukleären Zellen des peripheren Blutes eines von Zöliakie betroffenen Patienten inkubiert werden, erhalten mittels Zentrifugation, Rekuperation (280) und anschließender Suspension in Wachstumsmedium, das mit deaktiviertem Humanserum und mit Antibiotikum, das vorzugsweise Penicillin und/oder Streptomycin umfasst, versetzt wurde, und wobei die zelluläre Vitalität mittels eines Ausschlusstests mit Trypanblau bestimmt wird.

14. Verfahren nach Anspruch 13, weiterhin umfassend einen anschließenden Schritt der Isolierung und Aussaat von T-Lymphozyten in einer Vielzahl von Retentionsmitteln, die PBMC, das vorab bestrahlt ist, enthalten und als Zellen mit Antigen verwendet werden, und einen Schritt der anschließenden Zugabe des EV innerhalb der Rückhaltemittel; dem Schritt der Zugabe folgt ferner ein Inkubationsschritt (292) der Lymphozyten oder ein Stimulationsschritt mit dem polyklonalen Mitogen Phytohämagglutinin; wobei das Verfahren daher einen Inkubations- und anschließenden Sammel- und Einfrierschritt (293) des von den Rückhaltemitteln gesammelten Aufschwimmenden für eine anschließende Bestimmung des Interferon-γ-Spiegels umfasst und ferner einen Schritt des Pulsierens der Zellen T mit tritiiertem Thymidin (³H-Thymidin) umfasst.

15. Injizierbare Zubereitung zur Verwendung bei der Behandlung der Zöliakie, **dadurch gekennzeichnet, dass** es mittels extrazellulärer Vesikel (10) durch ein Realisierungsverfahren nach einem der Ansprüche 1 bis 14 realisiert wird.

## Revendications

1. Procédé de préparation d'une composition comprenant des vésicules extracellulaires (10) destinée au traitement de la maladie céliaque, ledit procédé étant **caractérisé en ce qu'**il comprend :
- une étape d'extraction des vésicules extracellulaires (10) de cellules souches mésenchymateuses (MSC) (5), provenant d'un échantillon unique de chorion (C) séparé à l'avance du placenta (200) dans une étape de séparation du chorion-placenta (93), et
- une étape (251, 252, 253) de stérilisation et d'élimination des protéines contaminantes d'un liquide semi-fini contenant lesdites vésicules extracellulaires (10), où ladite étape (251, 252, 253) de stérilisation et d'élimination des protéines contaminantes comprend au moins une première étape d'ultracentrifugation (252) dudit liquide semi-fini, et où
- ladite première étape d'ultracentrifugation (252) est effectuée suite à une phase de filtration (251) dudit liquide semi-fini ;
où ladite pluralité d'étapes de centrifugation (237-239) a lieu sur une partie surnageante d'un semi-fini supplémentaire comprenant lesdites cellules souches mésenchymateuses (5), ledit semi-fini supplémentaire se trouvant à l'avance incubé sur une période de temps s'étendant sur plusieurs jours et étant soumis à un traitement d'augmentation des propriétés immunologiques desdites cellules souches mésenchymateuses (5), ledit traitement d'augmentation des propriétés immunologiques desdites cellules souches mésenchymateuses (5) comprenant une étape de lavage à l'aide de PBS suivie de l'incubation avec de l'interféron-γ recombinant humain.

2. Procédé de préparation d'une composition selon la revendication 1, **caractérisé en ce qu'**il comprend une seconde étape d'ultracentrifugation (253) dudit liquide semi-fini, où ladite seconde étape d'ultracentrifugation (253) étant effectuée après ladite première étape d'ultracentrifugation (252) suite à une étape intermédiaire de lavage (252b), effectuée entre ladite première et ladite seconde étape d'ultracentrifugation (252, 253) et étant effectuée dans un milieu de croissance sans sérum.

3. Procédé de préparation d'une composition selon la revendication 1 ou 2, **caractérisé en ce qu'**il comprend une pluralité d'étapes de centrifugation (237-239), effectuées à l'avance par rapport à ladite première et/ou seconde étape d'ultracentrifugation (252, 253), où ladite pluralité d'étapes de centrifugation (237-239) sépare les détritus cellulaires et/ou les cellules mortes desdites vésicules extracellulaires (10), et où chacune desdites étapes de centrifugation (237-239) est effectuée sur une durée supérieure ou égale à 10 minutes, et où ledit liquide semi-fini comprend des éléments surnageants suite à ladite pluralité d'étapes de centrifugation.

4. Procédé selon la revendication 1, le surnageant nécessaire à la préparation des vésicules extracellulaires (10), qui représentent ledit semi-fini supplémentaire, sont soumises à une étape de changement d'un milieu de croissance depuis un premier type Chang C vers un second type milieu de Eagle modifié de Dulbecco, et où ledit second type de milieu de croissance est enrichi d'un pourcentage prédéfini d'exosome appauvri Exo-FBSTM.

5. Procédé selon l'une quelconque des revendications précédentes, dans lequel une pluralité d'échantillons dudit chorion (C) est soumise à une étape de digestion (141) effectuée en deux fois, en premier à l'aide de l'utilisation de pronase et par la suite de collagénase, en pourcentages préférablement et respectivement de 0,125 g/l et 0,1 g/l.

6. Procédé selon la revendication 5, dans lequel ladite étape de digestion (141) effectuée en deux fois est bloquée (142) à l'aide d'une addition d'une solution saline équilibrée et par la suite à travers une étape de centrifugation (143) ; et où suite au blocage de ladite seconde fois de l'étape de digestion chaque pellet produit à partir de ladite étape de digestion est en outre placé en suspension dans un milieu de croissance Chang C enrichi de sérum fœtal bovin désactivé à la chaleur et avec un antibiotique pour ensuite subir une étape ultérieure d'incubation sous une atmosphère humidifiée.

7. Procédé selon la revendication 6, dans lequel suite à ladite étape d'incubation en atmosphère humidifiée, le milieu de croissance est substitué et une étape de digestion enzymatique est effectuée après avoir retiré (147) le milieu de croissance et avoir ajouté de la trypsine.

8. Procédé selon la revendication 7, dans lequel ladite étape de digestion est bloquée (150) à l'aide de l'addition d'un pourcentage prédéfini de HBSS et à l'aide d'une centrifugation (151) suite à laquelle le pellet est placé en suspension dans un milieu de croissance et un contrôle de la vitalité des cellules est effectué.

9. Procédé selon l'une quelconque des revendications précédentes, comprenant une étape d'analyse de la stabilité génomique (190) où un échantillon de chaque lot de MSC est soumis à une analyse du caryotype comprenant une étape d'incubation (191) effectuée à l'avance par rapport à une étape de coloration pour faire apparaître des bandes, le « banding » (192).

10. Procédé selon l'une quelconque des revendications précédentes, comprenant une étape de test microbiologique pour vérifier l'absence de bactéries anaérobies et aérobies, d'endotoxines et de mycoplasmes.

11. Procédé selon l'une quelconque des revendications précédentes, dans lequel lesdites vésicules extracellulaires (10) obtenues suite à l'ultracentrifugation, sont placées pour adhérer à des boules (billes) ou des sphères de latex sulfaté et par la suite soumises à une analyse de cytofluorimétrie.

12. Procédé selon l'une quelconque des revendications précédentes, comprenant une étape d'évaluation de la composition de ladite préparation effectuée à l'aide d'une première étape de mise en suspension (260) d'un pellet (9) dans du paraformaldéhyde suivie d'une étape ultérieure de repos sous une atmosphère sèche pour permettre l'absorption de la membrane, ladite étape de repos étant en outre suivie d'une étape de mise en contraste (264) des échantillons avec de l'oxalate d'uranyle et d'incorporation ultérieure desdits échantillons dans un mélange d'acétate d'uranyle et de méthylcellulose.

13. Procédé selon l'une quelconque des revendications précédentes, comprenant une étape d'analyse « *in vitro* » de la réponse immunologique auxdites EV, comprenant une étape de collecte des échantillons d'EV d'un même lot qui sont incubées ensemble avec des cellules mononuclées provenant du sang périphérique d'un patient affecté de maladie céliaque, obtenues par centrifugation, récupération (280) et suspension ultérieure dans du milieu de croissance ajouté à du sérum humain désactivé et avec un antibiotique préférablement comprenant de la pénicilline et/ou de la streptomycine, et où la vitalité cellulaire est déterminée à l'aide d'un test d'exclusion avec du bleu de trypane.

14. Procédé selon la revendication 13, comprenant en outre une étape ultérieure d'isolement et d'ensemencement de lymphocytes T dans une pluralité de moyens de rétention à l'intérieur desquels sont contenues des PBMC à l'avance irradiées et utilisées comme cellules ayant de l'antigène, et une étape d'addition ultérieure desdites EV avec ledit moyen de rétention ; ladite étape d'addition étant en outre suivie d'une étape d'incubation (292) desdits lymphocytes ou d'une étape de stimulation avec de la phytohémagglutinine mitogène polyclonale ; ledit procédé comprenant par conséquent une étape d'incubation et de collecte ultérieure et de congélation (293) du surnageant recueilli dudit moyen de rétention pour une détermination ultérieure du niveau d'interféron-γ, et comprenant en outre une étape de pulsation des cellules T avec de la thymidine tritiée (thymidine ³H).

15. Préparation injectable pour l'utilisation dans le traitement de la maladie céliaque, **caractérisé en ce qu'**il est réalisé à l'aide de vésicules extracellulaires (10) à travers un procédé de réalisation selon l'une quelconque des revendications 1 à 14.
